# EUROPEAN PATENT SPECIFICATION

(11) **EP 3 262 168 B1**
(45) Date of publication and mention of the grant of the patent: **21.08.2019**
(21) Application number: 16710378.7
(22) Date of filing: 26.02.2016
(51) Int. Cl.: C12N 15/09

(54) **HOST CELL PROTEIN MODIFICATION**
WIRTSZELLENPROTEINMODIFIKATION
MODIFICATION DE PROTÉINES DE CELLULES HÔTES

(30) Priority: 27.02.2015 US 201562126213 P; 23.02.2016 US 201662298869 P
(43) Date of publication of application: 03.01.2018
(73) Proprietor: Regeneron Pharmaceuticals, Inc., Tarrytown, NY 10591 (US)
(72) Inventor: BURAKOV, Darya, Tarrytown, New York 10591 (US); GOREN, Michael, Tarrytown, New York 10591 (US); CHEN, Gang, Tarrytown, New York 10591 (US)
(74) Representative: Carpmaels & Ransford LLP
(86) International application number: PCT/US2016/019915
(87) International publication number: WO 2016/138467

(56) References cited:
- KIM DAESOO ET AL: "Phospholipase C isozymes selectively couple to specific neurotransmitter receptors", NATURE (LONDON), vol. 389, no. 6648, 1997, pages 290-293, XP002757066, ISSN: 0028-0836
- KURIAN MANJU A ET AL: "Phospholipase C beta 1 deficiency is associated with early-onset epileptic encephalopathy", BRAIN, vol. 133, no. Part 10, October 2010 (2010-10), pages 2964-2970, XP002757067,
- BOGARAPU SOUJANYA ET AL: "Phenotype of a Patient With Contiguous Deletion of TBX5 and TBX3: Expanding the Disease Spectrum", AMERICAN JOURNAL OF MEDICAL GENETICS, vol. 164, no. 5, May 2014 (2014-05), pages 1304-1309, XP002757068,
- BRUNTZ RONALD C ET AL: "Phospholipase D Signaling Pathways and Phosphatidic Acid as Therapeutic Targets in Cancer", PHARMACOLOGICAL REVIEWS, vol. 66, no. 4, October 2014 (2014-10), pages 1033-1079, XP002757069,

### SEQUENCE LISTING

<110> Regeneron Pharmaceuticals, Inc.
<120> HOST CELL PROTEIN MODIFICATION
<130> 10143WO01
<140> PCT Filed herewith
   <141> 2016-02-26
<150> US 62/126,213
   <151> 2015-02-27
<150> US 62/298,869
   <151> 2016-02-23
<160> 60
<170> PatentIn version 3.5
<210> 1
   <211> 17
   <212> PRT
   <213> Artificial Sequence
<220>
   <223> Synthetic
<400> 1
   Asp Leu Leu Val Ala His Asn Thr Trp Asn Ser Tyr Gln Asn Met Leu
   1               5                   10                  15
   Arg
<210> 2
   <211> 22
   <212> PRT
   <213> Artificial Sequence
<220>
   <223> Synthetic
<400> 2
   Leu Ile Arg Tyr Asn Asn Phe Leu His Asp Pro Leu Ser Leu Cys Glu
   1               5                   10                  15
   Ala Cys Ile Pro Lys Pro
               20
<210> 3
   <211> 12
   <212> PRT
   <213> Artificial Sequence
<220>
   <223> Synthetic
<400> 3
   Ser Val Leu Leu Asp Ala Ala Ser Gly Gln Leu Arg
   1               5                   10
<210> 4
   <211> 13
   <212> PRT
   <213> Artificial Sequence
<220>
   <223> Synthetic
<400> 4
   Asp Gln Ser Leu Val Glu Asp Met Asn Ser Met Val Arg
   1               5                   10
<210> 5
   <211> 17
   <212> PRT
   <213> Artificial Sequence
<220>
   <223> Synthetic
<400> 5
   Gln Phe Asn Ser Gly Thr Tyr Asn Asn Gln Trp Met Ile Val Asp Tyr
   1               5                   10                  15
   Lys
<210> 6
   <211> 20
   <212> PRT
   <213> Artificial Sequence
<220>
   <223> Synthetic
<400> 6
   Gln Gly Pro Gln Glu Ala Tyr Pro Leu Ile Ala Gly Asn Asn Leu Val
   1               5                   10                  15
   Phe Ser Ser Tyr
               20
<210> 7
   <211> 19
   <212> PRT
   <213> Artificial Sequence
<220>
   <223> Synthetic
<400> 7
   Ser Met Leu His Met Gly Gln Pro Asp Leu Trp Thr Phe Ser Pro Ile
   1               5                   10                  15
   Ser Val Pro
<210> 8
   <211> 21
   <212> PRT
   <213> Artificial Sequence
<220>
   <223> Synthetic
<400> 8
   Tyr Asn Asn Phe Leu His Asp Pro Leu Ser Leu Cys Glu Ala Cys Ile
   1               5                   10                  15
   Pro Lys Pro Asn Ala
               20
<210> 9
   <211> 13
   <212> PRT
   <213> Artificial Sequence
<220>
   <223> Synthetic
<400> 9
   Leu Ala Leu Asp Gly Ala Thr Trp Ala Asp Ile Phe Lys
   1               5                   10
<210> 10
   <211> 13
   <212> PRT
   <213> Artificial Sequence
<220>
   <223> Synthetic
<400> 10
   Leu Ser Leu Gly Ser Gly Ser Cys Ser Ala Ile Ile Lys
   1               5                   10
<210> 11
   <211> 13
   <212> PRT
   <213> Artificial Sequence
<220>
   <223> Synthetic
<400> 11
   Tyr Val Gln Pro Gln Gly Cys Val Leu Glu Trp Ile Arg
   1               5                   10
<210> 12
   <211> 19
   <212> PRT
   <213> Artificial Sequence
<220>
   <223> Synthetic
<400> 12
   Arg Met Ser Met Leu Ala Ala Ser Gly Pro Thr Trp Asp Gln Leu Pro
   1               5                   10                  15
   Pro Phe Gln
<210> 13
   <211> 12
   <212> PRT
   <213> Artificial Sequence
<220>
   <223> Synthetic
<400> 13
   Ser Phe Leu Glu Ile Asn Leu Glu Trp Met Gln Arg
   1               5                   10
<210> 14
   <211> 22
   <212> PRT
   <213> Artificial Sequence
<220>
   <223> Synthetic
<400> 14
   Val Leu Thr Ile Leu Glu Gln Ile Pro Gly Met Val Val Val Ala Asp
   1               5                   10                  15
   Ala Asp Lys Thr Glu Asp
               20
<210> 15
   <211> 14
   <212> PRT
   <213> Artificial Sequence
<220>
   <223> Synthetic
<400> 15
   Val Arg Ser Val Leu Leu Asp Ala Ala Ser Gly Gln Leu Arg
   1               5                   10
<210> 16
   <211> 16
   <212> PRT
   <213> Artificial Sequence
<220>
   <223> Synthetic
<400> 16
   Leu Thr Leu Leu Gln Leu Lys Gly Leu Glu Asp Ser Tyr Glu Gly Arg
   1               5                   10                  15
<210> 17
   <211> 18
   <212> PRT
   <213> Artificial Sequence
<220>
   <223> Synthetic
<400> 17
   Met Ser Met Leu Ala Ala Ser Gly Pro Thr Trp Asp Gln Leu Pro Pro
   1               5                   10                  15
   Phe Gln
<210> 18
   <211> 9
   <212> PRT
   <213> Artificial Sequence
<220>
   <223> Synthetic
<400> 18
   Val Thr Ser Phe Ser Leu Ala Lys Arg
   1               5
<210> 19
   <211> 9
   <212> PRT
   <213> Artificial Sequence
<220>
   <223> Synthetic
<400> 19
   Gln Asn Leu Asp Pro Pro Val Ser Arg
   1               5
<210> 20
   <211> 10
   <212> PRT
   <213> Artificial Sequence
<220>
   <223> Synthetic
<400> 20
   Ile Ile Lys Lys Tyr Gln Leu Gln Phe Arg
   1               5                   10
<210> 21
   <211> 19
   <212> PRT
   <213> Artificial Sequence
<220>
   <223> Synthetic
<400> 21
   Ala Gln Ile Phe Gln Arg Asp Gln Ser Leu Val Glu Asp Met Asn Ser
   1               5                   10                  15
   Met Val Arg
<210> 22
   <211> 22
   <212> PRT
   <213> Artificial Sequence
<220>
   <223> Synthetic
<400> 22
   Leu Ile Arg Tyr Asn Asn Phe Leu His Asp Pro Leu Ser Leu Cys Glu
   1               5                   10                  15
   Ala Cys Ile Pro Lys Pro
               20
<210> 23
   <211> 12
   <212> PRT
   <213> Artificial Sequence
<220>
   <223> Synthetic
<400> 23
   Ser Val Leu Leu Asp Ala Ala Ser Gly Gln Leu Arg
   1               5                   10
<210> 24
   <211> 13
   <212> PRT
   <213> Artificial Sequence
<220>
   <223> Synthetic
<400> 24
   Asp Gln Ser Leu Val Glu Asp Met Asn Ser Met Val Arg
   1               5                   10
<210> 25
   <211> 17
   <212> PRT
   <213> Artificial Sequence
<220>
   <223> Synthetic
<400> 25
   Asp Leu Leu Val Ala His Asn Thr Trp Asn Ser Tyr Gln Asn Met Leu
   1               5                   10                  15
   Arg
<210> 26
   <211> 21
   <212> PRT
   <213> Artificial Sequence
<220>
   <223> Synthetic
<400> 26
   Tyr Asn Asn Phe Leu His Asp Pro Leu Ser Leu Cys Glu Ala Cys Ile
   1               5                   10                  15
   Pro Lys Pro Asn Ala
               20
<210> 27
   <211> 19
   <212> PRT
   <213> Artificial Sequence
<220>
   <223> Synthetic
<400> 27
   Arg Met Ser Met Leu Ala Ala Ser Gly Pro Thr Trp Asp Gln Leu Pro
   1               5                   10                  15
   Pro Phe Gln
<210> 28
   <211> 19
   <212> PRT
   <213> Artificial Sequence
<220>
   <223> Synthetic
<400> 28
   Ser Met Leu His Met Gly Gln Pro Asp Leu Trp Thr Phe Ser Pro Ile
   1               5                   10                  15
   Ser Val Pro
<210> 29
   <211> 18
   <212> PRT
   <213> Artificial Sequence
<220>
   <223> Synthetic
<400> 29
   Met Ser Met Leu Ala Ala Ser Gly Pro Thr Trp Asp Gln Leu Pro Pro
   1               5                   10                  15
   Phe Gln
<210> 30
   <211> 14
   <212> PRT
   <213> Artificial Sequence
<220>
   <223> Synthetic
<400> 30
   Val Arg Ser Val Leu Leu Asp Ala Ala Ser Gly Gln Leu Arg
   1 5 10
<210> 31
   <211> 9
   <212> PRT
   <213> Artificial Sequence
<220>
   <223> Synthetic
<400> 31
   Gln Asn Leu Asp Pro Pro Val Ser Arg
   1 5
<210> 32
   <211> 585
   <212> PRT
   <213> Cricetulus griseus
<400> 32
   Met Ala Ala Pro Met Asp Arg Ser Pro Gly Gly Arg Ala Val Arg Ala
   1               5                   10                  15
   Leu Arg Leu Ala Leu Ala Leu Ala Ser Leu Thr Glu Val Leu Leu Asn
               20                  25                  30
   Cys Pro Ala Gly Ala Leu Pro Thr Gln Gly Pro Gly Arg Arg Arg Gln
           35                  40                  45
   Asn Leu Asp Pro Pro Val Ser Arg Val Arg Ser Val Leu Leu Asp Ala
       50                  55                  60
   Ala Ser Gly Gln Leu Arg Leu Val Asp Gly Ile His Pro Tyr Ala Val
   65                  70                  75                  80
   Ala Trp Ala Asn Leu Thr Asn Ala Ile Arg Glu Thr Gly Trp Ala Tyr
                   85                  90                  95
   Leu Asp Leu Gly Thr Asn Gly Ser Tyr Asn Asp Ser Leu Gln Ala Tyr
               100                 105                 110
   Ala Ala Gly Val Val Glu Ala Ser Val Ser Glu Glu Leu Ile Tyr Met
           115                 120                 125
   His Trp Met Asn Thr Met Val Asn Tyr Cys Gly Pro Phe Glu Tyr Glu
       130                 135                 140
   Val Gly Tyr Cys Glu Lys Leu Lys Ser Phe Leu Glu Ile Asn Leu Glu
   145                 150                 155                 160
   Trp Met Gln Arg Glu Met Glu Leu Ser Gln Asp Ser Pro Tyr Trp His
   165 170 175
   Gln Val Arg Leu Thr Leu Leu Gln Leu Lys Gly Leu Glu Asp Ser Tyr
   180 185 190
   Glu Gly Arg Leu Thr Phe Pro Thr Gly Arg Phe Thr Ile Lys Pro Leu
   195 200 205
   Gly Phe Leu Leu Leu Gln Ile Ala Gly Asp Leu Glu Asp Leu Glu Gln
   210 215 220
   Ala Leu Asn Lys Thr Ser Thr Lys Leu Ser Leu Gly Ser Gly Ser Cys
   225 230 235 240
   Ser Ala Ile Ile Lys Leu Leu Pro Gly Ala Arg Asp Leu Leu Val Ala
   245 250 255
   His Asn Thr Trp Asn Ser Tyr Gln Asn Met Leu Arg Ile Ile Lys Lys
   260 265 270
   Tyr Gln Leu Gln Phe Arg Gln Gly Pro Gln Glu Ala Tyr Pro Leu Ile
   275 280 285
   Ala Gly Asn Asn Leu Val Phe Ser Ser Tyr Pro Gly Thr Ile Phe Ser
   290 295 300
   Gly Asp Asp Phe Tyr Ile Leu Gly Ser Gly Leu Val Thr Leu Glu Thr
   305 310 315 320
   Thr Ile Gly Asn Lys Asn Pro Ala Leu Trp Lys Tyr Val Gln Pro Gln
   325 330 335
   Gly Cys Val Leu Glu Trp Ile Arg Asn Ile Val Ala Asn Arg Leu Ala
   340 345 350
   Leu Asp Gly Ala Thr Trp Ala Asp Ile Phe Lys Gln Phe Asn Ser Gly
   355 360 365
   Thr Tyr Asn Asn Gln Trp Met Ile Val Asp Tyr Lys Ala Phe Ile Pro
   370 375 380
   Asn Gly Pro Ser Pro Gly Ser Arg Val Leu Thr Ile Leu Glu Gln Ile
   385 390 395 400
   Pro Gly Met Val Val Val Ala Asp Lys Thr Glu Asp Leu Tyr Lys Thr
   405 410 415
   Thr Tyr Trp Ala Ser Tyr Asn Ile Pro Phe Phe Glu Ile Val Phe Asn
               420                 425                 430
   Ala Ser Gly Leu Gln Asp Leu Val Ala Gln Tyr Gly Asp Trp Phe Ser
           435                 440                 445
   Tyr Thr Lys Asn Pro Arg Ala Gln Ile Phe Gln Arg Asp Gln Ser Leu
       450                 455                 460
   Val Glu Asp Met Asn Ser Met Val Arg Leu Ile Arg Tyr Asn Asn Phe
   465                 470                 475                 480
   Leu His Asp Pro Leu Ser Leu Cys Glu Ala Cys Ile Pro Lys Pro Asn
                   485                 490                 495
   Ala Glu Asn Ala Ile Ser Ala Arg Ser Asp Leu Asn Pro Ala Asn Gly
               500                 505                 510
   Ser Tyr Pro Phe Gln Ala Leu Tyr Gln Arg Pro His Gly Gly Ile Asp
           515                 520                 525
   Val Lys Val Thr Ser Phe Ser Leu Ala Lys Arg Met Ser Met Leu Ala
       530                 535                 540
   Ala Ser Gly Pro Thr Trp Asp Gln Leu Pro Pro Phe Gln Trp Ser Leu
   545                 550                 555                 560
   Ser Pro Phe Arg Ser Met Leu His Met Gly Gln Pro Asp Leu Trp Thr
                   565                 570                 575
   Phe Ser Pro Ile Ser Val Pro Trp Asp
               580                 585
<210> 33
   <211> 2218
   <212> DNA
   <213> Cricetulus griseus
<400> 33
   gacagtcacg tggcccgact gaggcacgcg atggcggccc ccatggaccg gagccccggc      60
   ggccgggcgg tccgggcgct gaggctagcg ctggcgctgg cctcgctgac tgaggtgttg     120
   ctgaattgcc cggcgggcgc cctccccacg caggggcccg gcaggcggcg ccaaaacctc     180
   gacccgccgg tctcccgcgt ccgctcggtg ctgctggacg ccgcgtcggg tcagctgcgc     240
   ctggtggacg gcatccatcc ctacgcggtg gcctgggcca acctcaccaa cgccattcgc     300
   gagaccgggt gggcctatct ggacttgggt acaaatggaa gctacaatga cagcctgcag     360
   gcctatgcag ctggtgtggt ggaggcttct gtgtctgagg agctcatcta catgcactgg     420
   atgaacacaa tggtcaacta ctgtggcccc ttcgagtatg aagttggcta ctgtgagaag 480
   ctcaagagct tcctggagat caacctggag tggatgcaga gggagatgga actcagccag 540
   gactctccat attggcacca ggtgcggctg accctcctgc agctgaaagg cctagaggac 600
   agctacgaag gccgtttgac cttcccaact gggaggttca ccattaaacc cttggggttc 660
   ctcctgctgc agattgccgg agacctggaa gacctagagc aagccctgaa taagaccagc 720
   accaagcttt ccctgggctc cggttcctgc tccgctatca tcaagttgct gccaggcgca 780
   cgtgacctcc tggtggcaca caacacatgg aactcctacc agaacatgct acgcatcatc 840
   aagaagtacc agctgcagtt ccggcagggg cctcaagagg cgtaccccct gattgctggc 900
   aacaatttgg tcttttcgtc ttacccgggc accatcttct ctggcgatga cttctacatc 960
   ctgggcagtg ggctggtcac cctggagacc accattggca acaagaatcc agccctgtgg 1020
   aagtacgtgc agccccaggg ctgtgtgctg gagtggattc gaaacatcgt ggccaaccgc 1080
   ctggccttgg acggggccac ctgggcagac atcttcaagc agttcaatag tggcacgtat 1140
   aataaccaat ggatgattgt ggactacaag gcattcatcc ccaacgggcc cagccctgga 1200
   agccgagtgc ttaccatcct agaacagatc ccgggcatgg tggtggtggc cgacaagact 1260
   gaagatctct acaagacaac ctactgggct agctacaaca tcccgttctt tgagattgtg 1320
   ttcaacgcca gtgggctgca ggacttggtg gcccaatatg gagattggtt ttcctacact 1380
   aagaaccctc gagctcagat cttccagagg gaccagtcgc tggtggagga catgaattcc 1440
   atggtccggc tcataaggta caacaacttc cttcacgacc ctctgtcact gtgtgaagcc 1500
   tgtatcccga agcccaatgc agagaatgcc atctctgccc gctctgacct caatcctgcc 1560
   aatggctcct acccatttca agccctgtac cagcgtcccc acggtggcat cgatgtgaag 1620
   gtgaccagct tttcactggc caagcgcatg agcatgctgg cagccagtgg cccaacgtgg 1680
   gatcagttgc ccccattcca gtggagttta tcgccgttcc gcagcatgct tcacatgggc 1740
   cagcctgatc tctggacatt ctcacccatc agtgtcccat gggactgaga ctttgcctcc 1800
   acccagttgc cttcattctg tgtggccagt agggtcacac acctgctacc caccctttgg 1860
   ggctctgtcc tcactggact ctggtctgtg tggtctcctc tgcagggaca caaacccagt 1920
   aggctcagag ctgactccat ccccaagtct tctgccctcc atcactcctt ctctctgccc 1980
   ctgtcaccag tgggctgggg cttgtgcttg gctgtgggcc tggtgggatt ctgggcgcca 2040
   ttttcctagt gctggtccct cagtgtgtgt gtgggggaca ttgatagggc ttatcattgc 2100
   tgtcactact agcctgcggg cccatctcct cagggagcag tccatgtccc cttctctggg 2160
   cagctttcct gaggatagaa gcttgaaaac aaaaaaccaa agtttctggc tgctttta 2218
<210> 34
   <211> 594
   <212> PRT
   <213> Mus musculus
<400> 34
   Met Ala Ala Pro Val Asp Gly Ser Ser Gly Gly Trp Ala Ala Arg Ala
   1               5                   10                  15
   Leu Arg Arg Ala Leu Ala Leu Thr Ser Leu Thr Thr Leu Ala Leu Leu
               20                  25                  30
   Ala Ser Leu Thr Gly Leu Leu Leu Ser Gly Pro Ala Gly Ala Leu Pro
           35                  40                  45
   Thr Leu Gly Pro Gly Trp Gln Arg Gln Asn Pro Asp Pro Pro Val Ser
       50                  55                  60
   Arg Thr Arg Ser Leu Leu Leu Asp Ala Ala Ser Gly Gln Leu Arg Leu
   65                  70                  75                  80
   Glu Asp Gly Phe His Pro Asp Ala Val Ala Trp Ala Asn Leu Thr Asn
                   85                  90                  95
   Ala Ile Arg Glu Thr Gly Trp Ala Tyr Leu Asp Leu Ser Thr Asn Gly
               100                 105                 110
   Arg Tyr Asn Asp Ser Leu Gln Ala Tyr Ala Ala Gly Val Val Glu Ala
           115                 120                 125
   Ser Val Ser Glu Glu Leu Ile Tyr Met His Trp Met Asn Thr Val Val
       130                 135                 140
   Asn Tyr Cys Gly Pro Phe Glu Tyr Glu Val Gly Tyr Cys Glu Lys Leu
   145                 150                 155                 160
   Lys Asn Phe Leu Glu Ala Asn Leu Glu Trp Met Gln Arg Glu Met Glu
                   165                 170                 175
   Leu Asn Pro Asp Ser Pro Tyr Trp His Gln Val Arg Leu Thr Leu Leu
               180                 185                 190
   Gln Leu Lys Gly Leu Glu Asp Ser Tyr Glu Gly Arg Leu Thr Phe Pro
           195                 200                 205
   Thr Gly Arg Phe Thr Ile Lys Pro Leu Gly Phe Leu Leu Leu Gln Ile
       210                 215                 220
   Ser Gly Asp Leu Glu Asp Leu Glu Pro Ala Leu Asn Lys Thr Asn Thr
   225 230 235 240
   Lys Pro Ser Leu Gly Ser Gly Ser Cys Ser Ala Leu Ile Lys Leu Leu
   245 250 255
   Pro Gly Gly His Asp Leu Leu Val Ala His Asn Thr Trp Asn Ser Tyr
   260 265 270
   Gln Asn Met Leu Arg Ile Ile Lys Lys Tyr Arg Leu Gln Phe Arg Glu
   275 280 285
   Gly Pro Gln Glu Glu Tyr Pro Leu Val Ala Gly Asn Asn Leu Val Phe
   290 295 300
   Ser Ser Tyr Pro Gly Thr Ile Phe Ser Gly Asp Asp Phe Tyr Ile Leu
   305 310 315 320
   Gly Ser Gly Leu Val Thr Leu Glu Thr Thr Ile Gly Asn Lys Asn Pro
   325 330 335
   Ala Leu Trp Lys Tyr Val Gln Pro Gln Gly Cys Val Leu Glu Trp Ile
   340 345 350
   Arg Asn Val Val Ala Asn Arg Leu Ala Leu Asp Gly Ala Thr Trp Ala
   355 360 365
   Asp Val Phe Lys Arg Phe Asn Ser Gly Thr Tyr Asn Asn Gln Trp Met
   370 375 380
   Ile Val Asp Tyr Lys Ala Phe Leu Pro Asn Gly Pro Ser Pro Gly Ser
   385 390 395 400
   Arg Val Leu Thr Ile Leu Glu Gln Ile Pro Gly Met Val Val Val Ala
   405 410 415
   Asp Lys Thr Ala Glu Leu Tyr Lys Thr Thr Tyr Trp Ala Ser Tyr Asn
   420 425 430
   Ile Pro Tyr Phe Glu Thr Val Phe Asn Ala Ser Gly Leu Gln Ala Leu
   435 440 445
   Val Ala Gln Tyr Gly Asp Trp Phe Ser Tyr Thr Lys Asn Pro Arg Ala
   450 455 460
   Lys Ile Phe Gln Arg Asp Gln Ser Leu Val Glu Asp Met Asp Ala Met
   465 470 475 480
   Val Arg Leu Met Arg Tyr Asn Asp Phe Leu His Asp Pro Leu Ser Leu
   485 490 495
   Cys Glu Ala Cys Asn Pro Lys Pro Asn Ala Glu Asn Ala Ile Ser Ala
   500 505 510
   Arg Ser Asp Leu Asn Pro Ala Asn Gly Ser Tyr Pro Phe Gln Ala Leu
   515 520 525
   His Gln Arg Ala His Gly Gly Ile Asp Val Lys Val Thr Ser Phe Thr
   530 535 540
   Leu Ala Lys Tyr Met Ser Met Leu Ala Ala Ser Gly Pro Thr Trp Asp
   545 550 555 560
   Gln Cys Pro Pro Phe Gln Trp Ser Lys Ser Pro Phe His Ser Met Leu
   565 570 575
   His Met Gly Gln Pro Asp Leu Trp Met Phe Ser Pro Ile Arg Val Pro
   580 585 590
   Trp Asp
<210> 35
   <211> 585
   <212> PRT
   <213> Rattus norvegicus
<400> 35
   Met Ala Ala Pro Met Asp Arg Thr His Gly Gly Arg Ala Ala Arg Ala
   1 5 10 15
   Leu Arg Arg Ala Leu Ala Leu Ala Ser Leu Ala Gly Leu Leu Leu Ser
   20 25 30
   Gly Leu Ala Gly Ala Leu Pro Thr Leu Gly Pro Gly Trp Arg Arg Gln
   35 40 45
   Asn Pro Glu Pro Pro Ala Ser Arg Thr Arg Ser Leu Leu Leu Asp Ala
   50 55 60
   Ala Ser Gly Gln Leu Arg Leu Glu Tyr Gly Phe His Pro Asp Ala Val
   65 70 75 80
   Ala Trp Ala Asn Leu Thr Asn Ala Ile Arg Glu Thr Gly Trp Ala Tyr
   85 90 95
   Leu Asp Leu Gly Thr Asn Gly Ser Tyr Asn Asp Ser Leu Gln Ala Tyr
               100                 105                 110
   Ala Ala Gly Val Val Glu Ala Ser Val Ser Glu Glu Leu Ile Tyr Met
           115                 120                 125
   His Trp Met Asn Thr Val Val Asn Tyr Cys Gly Pro Phe Glu Tyr Glu
       130                 135                 140
   Val Gly Tyr Cys Glu Lys Leu Lys Ser Phe Leu Glu Ala Asn Leu Glu
   145                 150                 155                 160
   Trp Met Gln Arg Glu Met Glu Leu Ser Pro Asp Ser Pro Tyr Trp His
                   165                 170                 175
   Gln Val Arg Leu Thr Leu Leu Gln Leu Lys Gly Leu Glu Asp Ser Tyr
               180                 185                 190
   Glu Gly Arg Leu Thr Phe Pro Thr Gly Arg Phe Asn Ile Lys Pro Leu
           195                 200                 205
   Gly Phe Leu Leu Leu Gln Ile Ser Gly Asp Leu Glu Asp Leu Glu Pro
       210                 215                 220
   Ala Leu Asn Lys Thr Asn Thr Lys Pro Ser Val Gly Ser Gly Ser Cys
   225                 230                 235                 240
   Ser Ala Leu Ile Lys Leu Leu Pro Gly Ser His Asp Leu Leu Val Ala
                   245                 250                 255
   His Asn Thr Trp Asn Ser Tyr Gln Asn Met Leu Arg Ile Ile Lys Lys
               260                 265                 270
   Tyr Arg Leu Gln Phe Arg Glu Gly Pro Gln Glu Glu Tyr Pro Leu Ile
           275                 280                 285
   Ala Gly Asn Asn Leu Ile Phe Ser Ser Tyr Pro Gly Thr Ile Phe Ser
       290                 295                 300
   Gly Asp Asp Phe Tyr Ile Leu Gly Ser Gly Leu Val Thr Leu Glu Thr
   305                 310                 315                 320
   Thr Ile Gly Asn Lys Asn Pro Ala Leu Trp Lys Tyr Val Gln Pro Gln
                   325                 330                 335
   Gly Cys Val Leu Glu Trp Ile Arg Asn Ile Val Ala Asn Arg Leu Ala
               340                 345                 350
   Leu Asp Gly Ala Thr Trp Ala Asp Val Phe Arg Arg Phe Asn Ser Gly
   355 360 365
   Thr Tyr Asn Asn Gln Trp Met Ile Val Asp Tyr Lys Ala Phe Ile Pro
   370 375 380
   Asn Gly Pro Ser Pro Gly Ser Arg Val Leu Thr Ile Leu Glu Gln Ile
   385 390 395 400
   Pro Gly Met Val Val Val Ala Asp Lys Thr Ala Glu Leu Tyr Lys Thr
   405 410 415
   Thr Tyr Trp Ala Ser Tyr Asn Ile Pro Tyr Phe Glu Ser Val Phe Asn
   420 425 430
   Ala Ser Gly Leu Gln Ala Leu Val Ala Gln Tyr Gly Asp Trp Phe Ser
   435 440 445
   Tyr Thr Arg Asn Pro Arg Ala Lys Ile Phe Gln Arg Asp Gln Ser Leu
   450 455 460
   Val Glu Asp Val Asp Thr Met Val Arg Leu Met Arg Tyr Asn Asp Phe
   465 470 475 480
   Leu His Asp Pro Leu Ser Leu Cys Glu Ala Cys Ser Pro Lys Pro Asn
   485 490 495
   Ala Glu Asn Ala Ile Ser Ala Arg Ser Asp Leu Asn Pro Ala Asn Gly
   500 505 510
   Ser Tyr Pro Phe Gln Ala Leu Arg Gln Arg Ala His Gly Gly Ile Asp
   515 520 525
   Val Lys Val Thr Ser Val Ala Leu Ala Lys Tyr Met Ser Met Leu Ala
   530 535 540
   Ala Ser Gly Pro Thr Trp Asp Gln Leu Pro Pro Phe Gln Trp Ser Lys
   545 550 555 560
   Ser Pro Phe His Asn Met Leu His Met Gly Gln Pro Asp Leu Trp Met
   565 570 575
   Phe Ser Pro Val Lys Val Pro Trp Asp
   580 585
<210> 36
   <211> 589
   <212> PRT
   <213> Homo sapiens
<400> 36
   Met Val Gly Gln Met Tyr Cys Tyr Pro Gly Ser His Leu Ala Arg Ala
   1               5                   10                  15
   Leu Thr Arg Ala Leu Ala Leu Ala Leu Val Leu Ala Leu Leu Val Gly
               20                  25                  30
   Pro Phe Leu Ser Gly Leu Ala Gly Ala Ile Pro Ala Pro Gly Gly Arg
           35                  40                  45
   Trp Ala Arg Asp Gly Gln Val Pro Pro Ala Ser Arg Ser Arg Ser Val
       50                  55                  60
   Leu Leu Asp Val Ser Ala Gly Gln Leu Leu Met Val Asp Gly Arg His
   65                  70                  75                  80
   Pro Asp Ala Val Ala Trp Ala Asn Leu Thr Asn Ala Ile Arg Glu Thr
                   85                  90                  95
   Gly Trp Ala Phe Leu Glu Leu Gly Thr Ser Gly Gln Tyr Asn Asp Ser
               100                 105                 110
   Leu Gln Ala Tyr Ala Ala Gly Val Val Glu Ala Ala Val Ser Glu Glu
           115                 120                 125
   Leu Ile Tyr Met His Trp Met Asn Thr Val Val Asn Tyr Cys Gly Pro
       130                 135                 140
   Phe Glu Tyr Glu Val Gly Tyr Cys Glu Arg Leu Lys Ser Phe Leu Glu
   145                 150                 155                 160
   Ala Asn Leu Glu Trp Met Gln Glu Glu Met Glu Ser Asn Pro Asp Ser
                   165                 170                 175
   Pro Tyr Trp His Gln Val Arg Leu Thr Leu Leu Gln Leu Lys Gly Leu
               180                 185                 190
   Glu Asp Ser Tyr Glu Gly Arg Val Ser Phe Pro Ala Gly Lys Phe Thr
           195                 200                 205
   Ile Lys Pro Leu Gly Phe Leu Leu Leu Gln Leu Ser Gly Asp Leu Glu
       210                 215                 220
   Asp Leu Glu Leu Ala Leu Asn Lys Thr Lys Ile Lys Pro Ser Leu Gly
   225                 230                 235                 240
   Ser Gly Ser Cys Ser Ala Leu Ile Lys Leu Leu Pro Gly Gln Ser Asp
   245 250 255
   Leu Leu Val Ala His Asn Thr Trp Asn Asn Tyr Gln His Met Leu Arg
   260 265 270
   Val Ile Lys Lys Tyr Trp Leu Gln Phe Arg Glu Gly Pro Trp Gly Asp
   275 280 285
   Tyr Pro Leu Val Pro Gly Asn Lys Leu Val Phe Ser Ser Tyr Pro Gly
   290 295 300
   Thr Ile Phe Ser Cys Asp Asp Phe Tyr Ile Leu Gly Ser Gly Leu Val
   305 310 315 320
   Thr Leu Glu Thr Thr Ile Gly Asn Lys Asn Pro Ala Leu Trp Lys Tyr
   325 330 335
   Val Arg Pro Arg Gly Cys Val Leu Glu Trp Val Arg Asn Ile Val Ala
   340 345 350
   Asn Arg Leu Ala Ser Asp Gly Ala Thr Trp Ala Asp Ile Phe Lys Arg
   355 360 365
   Phe Asn Ser Gly Thr Tyr Asn Asn Gln Trp Met Ile Val Asp Tyr Lys
   370 375 380
   Ala Phe Ile Pro Gly Gly Pro Ser Pro Gly Ser Arg Val Leu Thr Ile
   385 390 395 400
   Leu Glu Gln Ile Pro Gly Met Val Val Val Ala Asp Lys Thr Ser Glu
   405 410 415
   Leu Tyr Gln Lys Thr Tyr Trp Ala Ser Tyr Asn Ile Pro Ser Phe Glu
   420 425 430
   Thr Val Phe Asn Ala Ser Gly Leu Gln Ala Leu Val Ala Gln Tyr Gly
   435 440 445
   Asp Trp Phe Ser Tyr Asp Gly Ser Pro Arg Ala Gln Ile Phe Arg Arg
   450 455 460
   Asn Gln Ser Leu Val Gln Asp Met Asp Ser Met Val Arg Leu Met Arg
   465 470 475 480
   Tyr Asn Asp Phe Leu His Asp Pro Leu Ser Leu Cys Lys Ala Cys Asn
   485 490 495
   Pro Gln Pro Asn Gly Glu Asn Ala Ile Ser Ala Arg Ser Asp Leu Asn
   500 505 510
   Pro Ala Asn Gly Ser Tyr Pro Phe Gln Ala Leu Arg Gln Arg Ser His
   515 520 525
   Gly Gly Ile Asp Val Lys Val Thr Ser Met Ser Leu Ala Arg Ile Leu
   530 535 540
   Ser Leu Leu Ala Ala Ser Gly Pro Thr Trp Asp Gln Val Pro Pro Phe
   545 550 555 560
   Gln Trp Ser Thr Ser Pro Phe Ser Gly Leu Leu His Met Gly Gln Pro
   565 570 575
   Asp Leu Trp Lys Phe Ala Pro Val Lys Val Ser Trp Asp
   580 585
<210> 37
   <211> 589
   <212> PRT
   <213> Bos taurus
<400> 37
   Met Val Ala Pro Met Tyr Gly Ser Pro Gly Gly Arg Leu Ala Arg Ala
   1               5                   10                  15
   Val Thr Arg Ala Leu Ala Leu Ala Leu Val Leu Ala Leu Leu Val Gly
               20                  25                  30
   Leu Phe Leu Ser Gly Leu Thr Gly Ala Ile Pro Thr Pro Arg Gly Gln
           35                  40                  45
   Arg Gly Arg Gly Met Pro Val Pro Pro Ala Ser Arg Cys Arg Ser Leu
       50                  55                  60
   Leu Leu Asp Pro Glu Thr Gly Gln Leu Arg Leu Val Asp Gly Arg His
   65                  70                  75                  80
   Pro Asp Ala Val Ala Trp Ala Asn Leu Thr Asn Ala Ile Arg Glu Thr
                   85                  90                  95
   Gly Trp Ala Phe Leu Glu Leu His Thr Asn Gly Arg Phe Asn Asp Ser
               100                 105                 110
   Leu Gln Ala Tyr Ala Ala Gly Val Val Glu Ala Ala Val Ser Glu Glu
               115                 120                 125
   Leu Ile Tyr Met Tyr Trp Met Asn Thr Val Val Asn Tyr Cys Gly Pro
       130                 135                 140
   Phe Glu Tyr Glu Val Gly Tyr Cys Glu Arg Leu Lys Asn Phe Leu Glu
   145                 150                 155                 160
   Ala Asn Leu Glu Trp Met Gln Lys Glu Met Glu Leu Asn Asn Gly Ser
                   165                 170                 175
   Ala Tyr Trp His Gln Val Arg Leu Thr Leu Leu Gln Leu Lys Gly Leu
               180                 185                 190
   Glu Asp Ser Tyr Glu Gly Ser Val Ala Phe Pro Thr Gly Lys Phe Thr
           195                 200                 205
   Val Lys Pro Leu Gly Phe Leu Leu Leu Gln Ile Ser Gly Asp Leu Glu
       210                 215                 220
   Asp Leu Glu Val Ala Leu Asn Lys Thr Lys Thr Asn His Ala Met Gly
   225                 230                 235                 240
   Ser Gly Ser Cys Ser Ala Leu Ile Lys Leu Leu Pro Gly Gln Arg Asp
                   245                 250                 255
   Leu Leu Val Ala His Asn Thr Trp His Ser Tyr Gln Tyr Met Leu Arg
               260                 265                 270
   Ile Met Lys Lys Tyr Trp Phe Gln Phe Arg Glu Gly Pro Gln Ala Glu
           275                 280                 285
   Ser Thr Arg Ala Pro Gly Asn Lys Val Ile Phe Ser Ser Tyr Pro Gly
       290                 295                 300
   Thr Ile Phe Ser Cys Asp Asp Phe Tyr Ile Leu Gly Ser Gly Leu Val
   305                 310                 315                 320
   Thr Leu Glu Thr Thr Ile Gly Asn Lys Asn Pro Ala Leu Trp Lys Tyr
                   325                 330                 335
   Val Gln Pro Thr Gly Cys Val Leu Glu Trp Met Arg Asn Val Val Ala
               340                 345                 350
   Asn Arg Leu Ala Leu Asp Gly Asp Ser Trp Ala Asp Ile Phe Lys Arg
           355                 360                 365
   Phe Asn Ser Gly Thr Tyr Asn Asn Gln Trp Met Ile Val Asp Tyr Lys
       370                 375                 380
   Ala Phe Val Pro Gly Gly Pro Ser Pro Gly Arg Arg Val Leu Thr Val
   385                 390                 395                 400
   Leu Glu Gln Ile Pro Gly Met Val Val Val Ala Asp Arg Thr Ser Glu
                   405                 410                 415
   Leu Tyr Gln Lys Thr Tyr Trp Ala Ser Tyr Asn Ile Pro Ser Phe Glu
               420                 425                 430
   Ser Val Phe Asn Ala Ser Gly Leu Pro Ala Leu Val Ala Arg Tyr Gly
           435                 440                 445
   Pro Trp Phe Ser Tyr Asp Gly Ser Pro Arg Ala Gln Ile Phe Arg Arg
       450                 455                 460
   Asn His Ser Leu Val His Asp Leu Asp Ser Met Met Arg Leu Met Arg
   465                 470                 475                 480
   Tyr Asn Asp Phe Leu His Asp Pro Leu Ser Leu Cys Lys Ala Cys Thr
                   485                 490                 495
   Pro Lys Pro Asn Gly Glu Asn Ala Ile Ser Ala Arg Ser Asp Leu Asn
               500                 505                 510
   Pro Ala Asn Gly Ser Tyr Pro Phe Gln Ala Leu His Gln Arg Ser His
           515                 520                 525
   Gly Gly Ile Asp Val Lys Val Thr Ser Thr Ala Leu Ala Lys Ala Leu
       530                 535                 540
   Arg Leu Leu Ala Val Ser Gly Pro Thr Trp Asp Gln Leu Pro Pro Phe
   545                 550                 555                 560
   Gln Trp Ser Thr Ser Pro Phe Ser Gly Met Leu His Met Gly Gln Pro
                   565                 570                 575
   Asp Leu Arg Lys Phe Ser Pro Ile Glu Val Ser Trp Asp
               580                 585
<210> 38
   <211> 30
   <212> DNA
   <213> Artificial Sequence
<220>
   <223> Synthetic
<400> 38
   ctgaggtgtt gctgaattgc ccggcgggcg 30
<210> 39
   <211> 30
   <212> DNA
   <213> Artificial Sequence
<220>
   <223> Synthetic
<400> 39
   gacgcggcgt ccagcagcac cgagcggacg 30
<210> 40
   <211> 30
   <212> DNA
   <213> Artificial Sequence
<220>
   <223> Synthetic
<400> 40
   acccgccggt ctcccgcgtc cgctcggtgc 30
<210> 41
   <211> 30
   <212> DNA
   <213> Artificial Sequence
<220>
   <223> Synthetic
<400> 41
   tggtggacgg catccatccc tacgcggtgg 30
<210> 42
   <211> 30
   <212> DNA
   <213> Artificial Sequence
<220>
   <223> Synthetic
<400> 42
   ggcggccccc atggaccgga gccccggcgg 30
<210> 43
   <211> 30
   <212> DNA
   <213> Artificial Sequence
<220>
   <223> Synthetic
<400> 43
   cccatggacc ggagccccgg cggccgggcg 30
<210> 44
   <211> 30
   <212> DNA
   <213> Artificial Sequence
<220>
   <223> Synthetic
<400> 44
   gacagtcacg tggcccgact gaggcacgcg 30
<210> 45
   <211> 20
   <212> DNA
   <213> Artificial Sequence
<220>
   <223> Synthetic
<400> 45
   gcccccatgg accggagccc 20
<210> 46
   <211> 20
   <212> DNA
   <213> Artificial Sequence
<220>
   <223> Synthetic
<400> 46
   tggaccggag ccccggcggc 20
<210> 47
   <211> 278
   <212> DNA
   <213> Cricetulus griseus
<400> 47
   ccggtctcgc gaatggcgtt ggtgaggttg gcccaggcca ccgcgtaggg atggatgccg 60
   tccaccaggc gcagctgacc cgacgcggcg tccagcagca ccgagcggac gcgggagacc 120
   ggcgggtcga ggttttggcg ccgcctgccg ggcccctgcg tggggagggc gcccgccggg 180
   caattcagca acacctcagt cagcgaggcc agcgccagcg ctagcctcag cgcccggacc 240
   gcccggccgc cggggctccg gtccatgggg gccgccat 278
<210> 48
   <211> 94
   <212> DNA
   <213> Cricetulus griseus
<400> 48
   ctcctcagac acagaagcct ccaccacacc agctgcatag gcctgcaggc tgtcattgta 60
   gcttccattt gtacccaagt ccagataggc ccac 94
<210> 49
   <211> 159
   <212> DNA
   <213> Cricetulus griseus
<400> 49
   ctggtgccaa tatggagagt cctggctgag ttccatctcc ctctgcatcc actccaggtt 60
   gatctccagg aagctcttga gcttctcaca gtagccaact tcatactcga aggggccaca 120
   gtagttgacc attgtgttca tccagtgcat gtagatgag 159
<210> 50
   <211> 101
   <212> DNA
   <213> Cricetulus griseus
<400> 50
   aggaacccca agggtttaat ggtgaacctc ccagttggga aggtcaaacg gccttcgtag 60
   ctgtcctcta ggcctttcag ctgcaggagg gtcagccgca c 101
<210> 51
   <211> 215
   <212> DNA
   <213> Cricetulus griseus
<400> 51
   cttgaggccc ctgccggaac tgcagctggt acttcttgat gatgcgtagc atgttctggt 60
   aggagttcca tgtgttgtgt gccaccagga ggtcacgtgc gcctggcagc aacttgatga 120
   tagcggagca ggaaccggag cccagggaaa gcttggtgct ggtcttattc agggcttgct 180
   ctaggtcttc caggtctccg gcaatctgca gcagg 215
<210> 52
   <211> 98
   <212> DNA
   <213> Cricetulus griseus
<400> 52
   cagcccactg cccaggatgt agaagtcatc gccagagaag atggtgcccg ggtaagacga 60
   aaagaccaaa ttgttgccag caatcagggg gtacgcct 98
<210> 53
   <211> 161
   <212> DNA
   <213> Cricetulus griseus
<400> 53
   gtgccactat tgaactgctt gaagatgtct gcccaggtgg ccccgtccaa ggccaggcgg 60
   ttggccacga tgtttcgaat ccactccagc acacagccct ggggctgcac gtacttccac 120
   agggctggat tcttgttgcc aatggtggtc tccagggtga c 161
<210> 54
   <211> 96
   <212> DNA
   <213> Cricetulus griseus
<400> 54
   gggatctgtt ctaggatggt aagcactcgg cttccagggc tgggcccgtt ggggatgaat 60
   gccttgtagt ccacaatcat ccattggtta ttatac 96
<210> 55
   <211> 72
   <212> DNA
   <213> Cricetulus griseus
<400> 55
   gggatgttgt agctagccca gtaggttgtc ttgtagagat cttcagtctt gtcggccacc 60
   accaccatgc cc 72
<210> 56
   <211> 153
   <212> DNA
   <213> Cricetulus griseus
<400> 56
   cttatgagcc ggaccatgga attcatgtcc tccaccagcg actggtccct ctggaagatc 60
   tgagctcgag ggttcttagt gtaggaaaac caatctccat attgggccac caagtcctgc 120
   agcccactgg cgttgaacac aatctcaaag aac 153
<210> 57
   <211> 163
   <212> DNA
   <213> Cricetulus griseus
<400> 57
   cttcacatcg atgccaccgt ggggacgctg gtacagggct tgaaatgggt aggagccatt 60
   ggcaggattg aggtcagagc gggcagagat ggcattctct gcattgggct tcgggataca 120
   ggcttcacac agtgacagag ggtcgtgaag gaagttgttg tac 163
<210> 58
   <211> 168
   <212> DNA
   <213> Cricetulus griseus
<400> 58
   tcagtcccat gggacactga tgggtgagaa tgtccagaga tcaggctggc ccatgtgaag 60
   catgctgcgg aacggcgata aactccactg gaatgggggc aactgatccc acgttgggcc 120
   actggctgcc agcatgctca tgcgcttggc cagtgaaaag ctggtcac 168
<210> 59
   <211> 20
   <212> RNA
   <213> Artificial Sequence
<220>
   <223> Synthetic
<400> 59
   gggcuccggu ccaugggggc 20
<210> 60
   <211> 20
   <212> RNA
   <213> Artificial Sequence
<220>
   <223> Synthetic
<400> 60
   gccgccgggg cuccggucca 20

## Description

### Sequence Listing

This application includes a sequence listing in computer readable form in a file named 10143US01_ST25.txt created on February 26, 2016 (41,768 bytes), which is incorporated by reference herein.

### Field of the Invention

The invention provides for cells and methods for expression and purification of recombinant proteins in eukaryotic cells. In particular, the invention includes methods and compositions for expression of proteins in eukaryotic cells, particularly Chinese hamster (*Cricetulus griseus*) cell lines, that employ downregulating gene expression of endogenous proteins in order to control production of such unwanted "sticky" host cell proteins. The invention includes polynucleotides and modified cells that facilitate purification of an exogenous recombinant protein of interest. The methods of the invention efficiently target host cell proteins in the Chinese hamster cellular genome in order to facilitate enhanced and stable expression of recombinant proteins expressed by the modified cells.

### Background

Cellular expression systems aim to provide a reliable and efficient source for the manufacture of biopharmaceutical products for therapeutic use. Purification of any recombinant protein produced by either eukaryotic or prokaryotic cells in such systems is an ongoing challenge due to, for example, the plethora of host cell proteins and nucleic acid molecules that need to be eliminated from the final pharmaceutical grade product.

Certain dynamics of host cell proteins, viewed as impure byproducts, have been surveyed during various stages of bioprocessing. Advanced liquid chromatography/mass spectrometry (LC/MS) was done to detect and monitor *E. coli* HCPs accompanying peptibodies produced by cell culture (Schenauer, MR., et al, 2013, Biotechnol Prog 29(4):951-7). The information obtained by HCP profiles is useful for monitoring process development and assessing quality and purity of the product in order to assess safety risks posed by any one or more HCP(s).

Changes in cell culture conditions of eukaryotic cells has been shown to impact the purity of manufactured proteins, as seen by the increased quantity of HCPs of CHO cells upon downstream bioprocessing alterations (Tait, et al, 2013, Biotechnol Prog 29(3):688-696). The detrimental effect of leftover HCPs in any product may affect the overall quality or quantity, or both the quality and quantity of the product. Current protocols seek to alter the protein of interest produced by the cell (*e.g*. therapeutic antibody) to eliminate differential binding or interaction with the protein of interest and the host cell protein (Zhang, Q. et al, mAbs, Published online: 11 Feb 2014).

Despite the availability of numerous cell expression systems, engineered cell lines and systems that do not negatively impact the biological properties of an expressed protein of interest are particularly advantageous. Accordingly, there is a need in the art for improved methods towards preparation of quality protein samples for downstream bioprocessing and subsequently commercial use.

### BRIEF SUMMARY

The use of gene editing tools to eliminate a contaminant host cell protein is contemplated, and thus, engineered host cells for more efficient manufacturing processing of proteins is provided.

In one aspect, the invention provides a recombinant host cell, wherein the cell is modified to decrease the expression levels of esterase relative to the expression levels of esterase in an unmodified cell.

In another aspect, the invention provides a recombinant host cell, wherein the cell is modified to have no expression of a target esterase.

In some embodiments, the esterase is a phospholipase, particularly a phospholipase B-like protein. In further embodiments, the phospholipase is a phospholipase B-like 2 protein.

In some embodiments, a gene of interest is exogenously added to the recombinant host cell. In other embodiments, the exogenously added gene encodes a protein of interest (POI), for example the POI is selected form the group consisting of antibody heavy chain, antibody light chain, antigen-binding fragment, and Fc-fusion protein.

The invention provides a cell comprising a nonfunctional PLBD2 protein.

The invention provides making a cell by PLBD2 target disruption. In some embodiments, the method comprises a site-specific nuclease for disrupting or editing the cell genome at a target site or sequence. In some embodiments, the PLBD2 target site comprises a position within SEQ ID NO:33 or adjacent to a position within SEQ ID NO:33 selected from the group consisting of nucleotides spanning positions numbered 1-20, 10-30, 20-40, 30-50, 30-60, 30-70, 40-60, 40-70, 50-70, 60-80, 70-90, 80-100, 90-110, 110-140, 120-140, 130-150, 140-160, 150-170, 160-180, 160-180, 170-190, 180-200, 180-220, 190-230, 190-210, 200-220, 210-230, 220-240, 230-250, 240-260, and 250-270 of SEQ ID NO:33.

In another embodiment, the target site at a position within SEQ ID NO:33 or adjacent to a position within SEQ ID NO:33 is selected from the group consisting of nucleotides spanning positions numbered 37-56, 44-56, 33-62, 40-69, 110-139, 198-227, 182-211, and 242-271 of SEQ ID NO:33. In this regard, the PLBD2 target site is partially or fully within or encompassed by the nucleotide positions of SEQ ID NO:33 provided herein, and disrupting or editing the cell genome at the target site or sequence may consist of deleting or inserting one or more nucleotides within the nucleotide positions of SEQ ID NO:33 provided herein, whereas disrupting or editing alters a subsequent transcript as compared to that transcribed from a wild-type cell (i.e. a cell free of genomic disruption or gene editing). In some embodiments, the subsequent transcript of an altered gene results in a frameshift of the translated protein. In some embodiments, the subsequent transcript of an altered gene results in an altered protein that is subject to degradation, is not detectable by a standard method such as mass spectrometry, or has no detectable activity. In some embodiments, the targeted disruption or editing occurs on both alleles of the gene (*i.e*. biallelic disruption or biallelic alteration).

In certain embodiments, the cell further integrates an exogenous nucleic acid sequence. In other embodiments, the cell is capable of producing an exogenous protein of interest. In still other embodiments, the altered protein resulting from a disrupted gene does not bind to the protein of interest produced by the cell.

In another aspect, an isolated Chinese hamster ovary (CHO) cell is provided that comprises an engineered nucleic acid sequence comprising a variant of the PLBD2 gene (such as a variant of SEQ ID NO:33). In one embodiment, the PLBD2 gene comprises GACAGTCACG TGGCCCGACT GAGGCACGCG, nucleotides 1-30 of SEQ ID NO:33 (SEQ ID NO: 44). In another embodiment, the PLBD2 gene is engineered to disrupt expression of the open reading frame. In other embodiments, the invention provides an isolated CHO cell comprising (a) a disrupted PLBD2 gene comprising GACAGTCACG TGGCCCGACT GAGGCACGCG (SEQ ID NO: 44, also nucleotides 1-30 of SEQ ID NO:33), (b) a disrupted esterase gene comprising a nucleotide encoding any one of the amino acid sequences in Table 1, or (c) a protein fragment of Table 1 expressed by a disrupted PLBD2 gene; and an exogenous nucleic acid sequence comprising a gene of interest.

In another aspect, a method of producing a protein of interest using a recombinant host cell is provided, wherein the host cell is modified to decrease the expression levels of esterase relative to the expression levels of esterase in an unmodified cell.

In another embodiment, the method comprises the modified host cell having decreased esterase expression and an exogenous nucleic acid sequence comprising a gene of interest (GOI).

In certain embodiments, the exogenous nucleic acid sequence comprises one or more genes of interest. In some embodiments, the one or more genes of interest are selected from the group consisting of a first GOI, a second GOI and a third GOI.

In another aspect, the invention provides expression systems comprising the recombinant host cell comprising modified or nonfunctional esterase.

In yet another embodiment, the cell comprises a GOI operably linked to a promoter capable of driving expression of the GOI, wherein the promoter comprises a eukaryotic promoter that can be regulated by an activator or inhibitor. In other embodiments, the eukaryotic promoter is operably linked to a prokaryotic operator, and the eukaryotic cell optionally further comprises a prokaryotic repressor protein.

In another embodiment, one or more selectable markers are expressed by the modified host cell. In some embodiments, the genes of interest and/or the one or more selectable markers are operably linked to a promoter, wherein the promoter may be the same or different. In another embodiment, the promoter comprises a eukaryotic promoter (such as, for example, a CMV promoter or an SV40 late promoter), optionally controlled by a prokaryotic operator (such as, for example, a tet operator). In other embodiments, the cell further comprises a gene encoding a prokaryotic repressor (such as, for example, a tet repressor).

In one aspect, a CHO host cell is provided, comprising recombinase recognition sites. In some embodiments, the recombinase recognition sites are selected from a LoxP site, a Lox511 site, a *Lox*2272 site, *Lox*2372, *Lox*5171, and a frt site.

In another embodiment, the cell further comprises a gene capable of expressing a recombinase. In some embodiments, the recombinase is a Cre recombinase.

In one embodiment, the selectable marker gene is a drug resistance gene. In another embodiment, the drug resistance gene is a neomycin resistance gene or a hygromycin resistance gene. In another embodiment, the second and third selectable marker genes encode two different fluorescent proteins. In one embodiment, the two different fluorescent proteins are selected from the group consisting of *Discosoma* coral (DsRed), green fluorescent protein (GFP), enhanced green fluorescent protein (eGFP), cyano fluorescent protein (CFP), enhanced cyano fluorescent protein (eCFP), yellow fluorescent protein (YFP), enhanced yellow fluorescent protein (eYFP) and far-red fluorescent protein (mKate).

In one embodiment, the first, second, and third promoters are the same. In another embodiment, the first, second, and third promoters are different from each other. In another embodiment, the first promoter is different from the second and third promoters, and the second and third promoters are the same. In more embodiments, the first promoter is an SV40 late promoter, and the second and third promoters are each a human CMV promoter. In other embodiments, the first and second promoters are operably linked to a prokaryotic operator.

In one embodiment, the host cell line has an exogenously added gene encoding a recombinase integrated into its genome, operably linked to a promoter. In another embodiment, the recombinase is Cre recombinase. In another embodiment, the host cell has a gene encoding a regulatory protein integrated into its genome, operably linked to a promoter. In more embodiments, the regulatory protein is a tet repressor protein.

In one embodiment, the first GOI and the second GOI encode a light chain, or fragment thereof, of an antibody or a heavy chain, or fragment thereof, of an antibody. In another embodiment, the first GOI encodes a light chain of an antibody and the second GOI encodes a heavy chain of an antibody.

In certain embodiments, the first, second and third GOI encode a polypeptide selected from the group consisting of a first light chain, or fragment thereof, a second light chain, or fragment thereof and a heavy chain, or fragment thereof. In yet another embodiment, the first, second and third GOI encode a polypeptide selected from the group consisting of a light chain, or fragment thereof, a first heavy chain, or fragment thereof and a second heavy chain, or fragment thereof.

In one aspect, a method is provided for making a protein of interest, comprising (a) introducing into a CHO host cell a gene of interest (GOI), wherein the GOI integrates into a specific locus such as a locus described in US Patent No. 7771997B2, issued August 10, 2010 or other stable integration and/or expression-enhancing locus; (b) culturing the cell of (a) under conditions that allow expression of the GOI; and (c) recovering the protein of interest. In one embodiment, the protein of interest is selected from the group consisting of a subunit of an immunoglobulin, or fragment thereof, and a receptor, or ligand-binding fragment thereof. In certain embodiments, the protein of interest is selected from the group consisting of an antibody light chain, or antigen-binding fragment thereof, and an antibody heavy chain, or antigen-binding fragment thereof.

In certain embodiments, the CHO host cell genome comprises further modifications, and comprises one or more recombinase recognition sites as described above, and the GOI is introduced into a specific locus through the action of a recombinase that recognizes the recombinase recognition site.

In some embodiments, the GOI is introduced into the cell employing a targeting vector for recombinase-mediated cassette exchange (RMCE) when the CHO host cell genome comprises at least one exogenous recognition sequence within a specific locus.

In another embodiment, the GOI is introduced into the cell employing a targeting vector for homologous recombination, and wherein the targeting vector comprises a 5' homology arm homologous to a sequence present in the specific locus, a GOI, and a 3' homology arm homologous to a sequence present in the specific locus. In another embodiment, the targeting vector further comprises two, three, four, or five or more genes of interest. In another embodiment, one or more of the genes of interest are operably linked to a promoter.

In another aspect, a method is provided for modifying a CHO cell genome to integrate an exogenous nucleic acid sequence, comprising the step of introducing into the cell a vehicle comprising an exogenous nucleic acid sequence wherein the exogenous nucleic acid integrates within a locus of the genome.

In yet another aspect, the invention provides a process for manufacturing a stable protein formulation comprising the steps of: (a) extracting a protein fraction from the modified host cell of the invention having decreased or ablated expression of esterase, (b) contacting the protein fraction comprising a protein of interest with a column selected from the group consisting of protein A affinity (PA), cation exchange (CEX) and anion exchange (AEX) chromatography, (c) collecting the protein of interest from the media, wherein a reduced level of the esterase activity is associated with the protein fraction collected at step (c), thus providing a stable protein formulation.

In yet another aspect, the invention provides a process for reducing esterase activity in a protein formulation comprising the steps of: (a) modifying a host cell to decrease or ablate expression of esterase, (b) transfecting the host cell with a protein of interest, (c) extracting a protein fraction from the modified host cell, (c) contacting the protein fraction comprising the protein of interest with a column selected from the group consisting of protein A affinity (PA), cation exchange (CEX) and anion exchange (AEX) chromatography, (d) collecting the protein of interest from the media, and (e) combining the protein of interest with a fatty acid ester, and optionally a buffer and thermal stabilizer, thus providing a protein formulation essentially free of detectable esterase activity. In some embodiments, the protein formulation is essentially free of PLBD2 protein or PLBD2 activity.

In yet another aspect, a method is provided for modifying a CHO cell genome to express a therapeutic agent comprising a vehicle for introducing, into the genome, an exogenous nucleic acid comprising a sequence for expression of the therapeutic agent, wherein the vehicle comprises a 5' homology arm homologous to a sequence present in the nucleotide sequence of SEQ ID NO:33, a nucleic acid encoding the therapeutic agent, and a 3' homology arm homologous to a sequence present in the nucleotide sequence of SEQ ID NO:33.

In one more aspect, the invention provides a modified CHO host cell comprising a modified CHO genome wherein the CHO genome is modified by disruption of target sequence within a nucleotide sequence at least 90% identical to SEQ ID NO: 33.

In another aspect, the invention provides a modified eukaryotic host cell comprising a modified eukaryotic genome wherein the eukaryotic genome is modified at a target sequence in a coding region of the PLBD2 gene by a site-specific nuclease. In some embodiments, the site-specific nuclease comprises a zinc finger nuclease (ZFN), a ZFN dimer, a transcription activator-like effector nuclease (TALEN), a TAL effector domain fusion protein, or an RNA-guided DNA endonuclease. The invention also provides methods of making such a modified eukaryotic host cell.

In any of the aspects and embodiments described above, the target sequence can be placed in the indicated orientation as in SEQ ID NO:33, or in the reverse of the orientation of SEQ ID NO:33.

In another aspect, a recombinant host cell comprising an altered PLBD2 gene is provided. In some embodiments, a recombinant host cell is provided wherein the PLBD2 gene is altered by disruption of a coding region. In other embodiments, the disrupted coding region is within the exon nucleotide sequence selected from the group consisting of Exon 1, Exon 2, Exon 3, Exon 4, Exon 5, Exon 6, Exon 7, Exon 8, Exon 9, Exon 10, Exon 11, and Exon 12. In certain embodiments, the disrupted coding region is within the exon nucleotide sequence selected from the group consisting of Exon 1 and Exon 2.

In one embodiment, a recombinant host cell is provided wherein the PLBD2 gene alteration comprises a biallelic alteration.

In another embodiment, a recombinant host cell is provided wherein the PLBD2 gene alteration comprises a deletion of 1 or more base pairs, 2 or more base pairs, 3 or more base pairs, 4 or more base pairs, 5 or more base pairs, 6 or more base pairs, 7 or more base pairs, 8 or more base pairs, 9 or more base pairs, 10 or more base pairs, 11 or more base pairs, 12 or more base pairs, 13 or more base pairs, 14 or more base pairs, 15 or more base pairs, 16 or more base pairs, 17 or more base pairs, 18 or more base pairs, 19 or more base pairs, or 20 or more base pairs.

Any of the aspects and embodiments of the invention can be used in conjunction with any other aspect or embodiment of the invention, unless otherwise specified or apparent from the context.

Other objects and advantages will become apparent from a review of the ensuing detailed description.

### BRIEF DESCRIPTION OF THE DRAWINGS

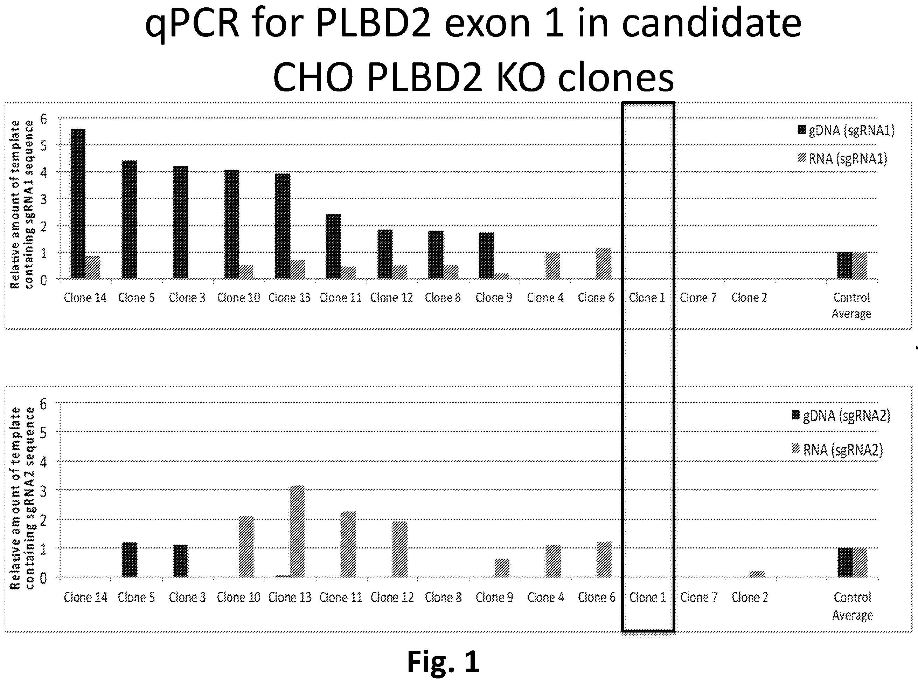

**Fig. 1** depicts the results of Taqman® quantitative polymerase chain reaction (qPCR) experiments to detect genomic (gDNA) or transcripts (mRNA) of the modified clones. Primers and probes were designed to flank the sequences predicted as subject to targeted disruption within exon 1, either starting at nucleotide 37 (sgRNA1) or starting at nucleotide 44 (sgRNA2) of SEQ ID NO:33. Relative amount of amplicons from clones targeted by either sgRNA1 or sgRNA2 are graphed (*i.e*. relative to amplicons derived from the negative control transfection clones which were subject to no sgRNA or unmatched sgRNA). Clone 1, for example, has relatively no amplified gDNA nor mRNA per qPCR of the targeted exon 1 region. Clone 1 and several other clones were selected for follow up analysis.

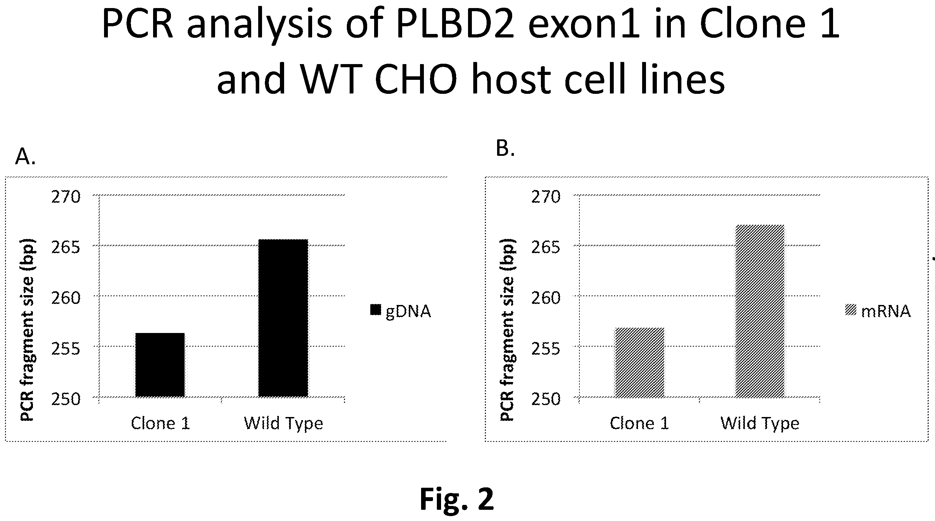

**Fig. 2A** and **Fig. 2B** illustrate the results of further PCR analysis of a Clone 1 cells population compared to wild type Chinese hamster overy (CHO) cells. **Fig. 2A** shows a PCR amplicon from Clone 1 that is shorter in length (bp) compared to the amplicon from genomic DNA of wild type cells. **Fig. 2B** shows a PCR amplicon from Clone 1 that is shorter in length (bp) compared to the amplicon from mRNA of wild type cells. Sequencing confirmed an 11bp deletion in the PLBD2 gene of Clone 1.

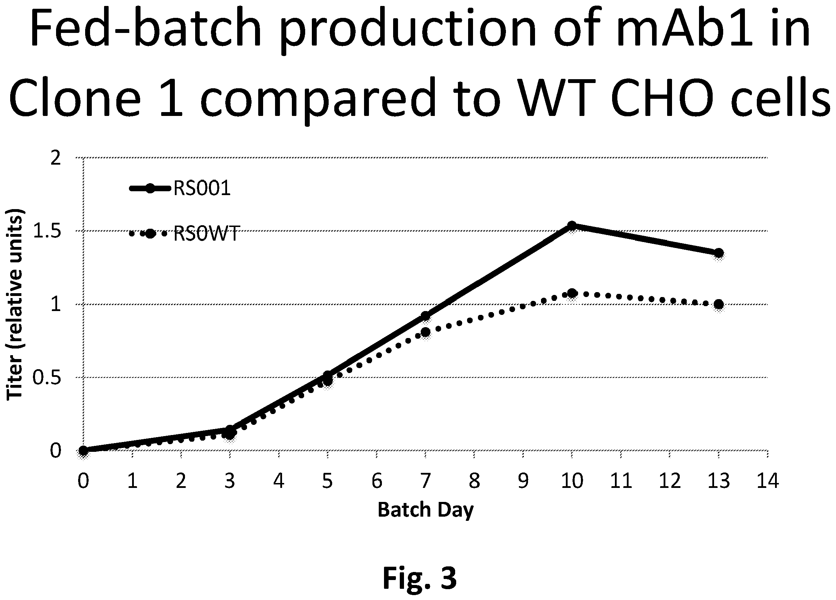

**Fig. 3** illustrates the relative protein titer of monoclonal antibody 1 (mAb1)-expressing Clone 1 cells (RS001) or mAb1-expressing wild type CHO cells (RS0WT) subject to the same fed-batch culture conditions for 12 days. Samples of conditioned medium were extracted for each culture, and the Protein A binding fraction was quantified at Day 0, 3, 5, 7, 10, and 13.

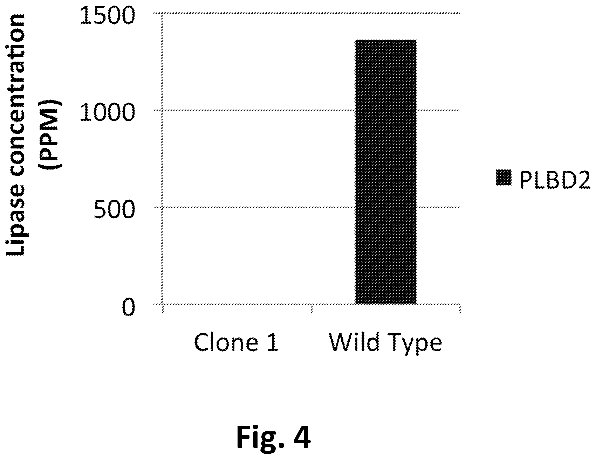

**Fig. 4** shows the results of RS001 or RS0WT cells following production culture and protein purification using either Protein A (PA) alone, or PA and anion exchange (AEX) chromatography. PA-purified mAb1 from RS001 and RS0WT was analyzed for lipase abundance using trypsin digest mass spectrometry. As such, trypsin digests of RS001- and RS0WT-produced mAb1 were injected into a reverse phase liquid chromatography column coupled to a triple quadrupole mass spectrometer set to monitor a specific PLBD2 product fragment (as in Table 1). Control reactions containing reference samples of mAb1 (with no endogenous PLBD2) spiked with varying amounts of recombinant PLBD2 were also analyzed and plotted. The signals detected in the experiments were compared to the control reactions to determine concentration of PLBD2. mAb1 produced from Clone 1 shows no detectable amounts of PLBD2 when purified with PA alone.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure.

Unless defined otherwise, or otherwise specified, all technical and scientific terms used herein have the same meaning as commonly understood by one of ordinary skill in the art to which this invention belongs.

Although any methods and materials similar or equivalent to those described herein can be used in the practice or testing of the present invention, particular methods and materials are now described. All publications mentioned herein are incorporated herein by reference in their entirety.

### Definitions

The phrase "exogenously added gene" or "exogenously added nucleic acid" refers to any DNA sequence or gene not present within the genome of the cell as found in nature. For example, an "exogenously added gene" within a CHO genome, can be a gene from any other species (*e.g*., a human gene), a chimeric gene (*e.g*., human/mouse), or a hamster gene not found in nature within the particular CHO locus in which the gene is inserted (*i.e*., a hamster gene from another locus in the hamster genome), or any other gene not found in nature to exist within a CHO locus of interest.

Percent identity, when describing an esterase, *e.g*. a phospholipase protein or gene, such as SEQ ID NO:32 or SEQ ID NO:33, respectively, is meant to include homologous sequences that display the recited identity along regions of contiguous homology, but the presence of gaps, deletions, or insertions that have no homolog in the compared sequence are not taken into account in calculating percent identity.

A "percent identity" determination between, *e.g*., SEQ ID NO:32 with a species homolog would not include a comparison of sequences where the species homolog has no homologous sequence to compare in an alignment (*i.e*., SEQ ID NO:32 compared to a fragment thereof, or the species homolog has a gap or deletion, as the case may be). Thus, "percent identity" does not include penalties for gaps, deletions, and insertions.

"Targeted disruption" of a gene or nucleic acid sequence refers to gene targeting methods that direct cleavage or breaks (such as double stranded breaks) in genomic DNA and thus cause a modification to the coding sequence of such gene or nucleic acid sequence. Gene target sites are the sites selected for cleavage or break by a nuclease. The DNA break is normally repaired by the non-homologous end-joining (NHEJ) DNA repair pathway. During NHEJ repair, insertions or deletions (InDels) may occur, as such, a small number of nucleotides are either inserted or deleted at random at the site of the break and these InDels may shift or disrupt the open reading frame (ORF) of the target gene. Shifts in the ORF may cause significant changes in the resulting amino acid sequence downstream of the DNA break, or may introduce a premature stop codon, therefore the expressed protein, if any, is rendered nonfunctional or subject to degradation.

"Targeted insertion" refers to gene targeting methods employed to direct insertion or integration of a gene or nucleic acid sequence to a specific location on the genome, *i.e*., to direct the DNA to a specific site between two nucleotides in a contiguous polynucleotide chain. Targeted insertion may also be performed to introduce a small number of nucleotides or to introduce an entire gene cassette, which includes multiple genes, regulatory elements, and/or nucleic acid sequences. "Insertion" and "integration" are used interchangeably.

"Recognition site" or "recognition sequence" is a specific DNA sequence recognized by a nuclease or other enzyme to bind and direct site-specific cleavage of the DNA backbone. Endonucleases cleave DNA within a DNA molecule. Recognition sites are also referred to in the art as recognition target sites.

Polysorbates are fatty acid esters of sorbitan or iso-sorbide (polyoxyethylene sorbitan or iso-sorbide mono- or di- esters). The polyoxyethylene serves as the hydrophilic head group and the fatty acid as the lipophilic hydrophobic tail. The effectiveness as a surfactant of the polysorbate depends upon the amphiphilic nature of the molecule with both hydrophilic head and hydrophobic tail present (in a single molecule). When a polysorbate degrades (hydrolyzes) into its component head group and fatty acid tail, it loses its effectiveness as a protein stabilizer, potentially allowing for aggregation and subsequent subvisible particle (SVP) formation is an indicator of such degradation. SVPs may attribute to immunogenicity. Regulatory authorities like the United States Food and Drug Administration (USFDA) provide limitations on the number of subvisible particles (SVPs) allowed in a pharmaceutical formulation. United States Pharmacopeia (USP) publishes standards for strength, purity and quality of drugs and drug ingredients, as well as food ingredients and dietary supplements.

The phrase "esterase activity" refers to the enzymatic activity of a hydrolase enzyme that cleaves (hydrolyzes) esters, such as fatty acid-esters, into acids (i.e. free fatty acids) and alcohols (i.e. ester-containing compounds).

Protein A-binding fraction refers to the fraction of cell lysate from cultured cells expressing a protein of interest which binds to a Protein A affinity format. It is well understood in the art that Protein A affinity chromatography, such as Protein A chromatography medium, such as resins, beads, columns and the like, are utilized to capture Fc-containing proteins due to their affinity to Protein A.

Phospholipase B-like 2 (PLBD2) refers to the homologs of a phospholipase gene known as NCBI RefSeq. XM_003510812.2 (SEQ ID NO:33) or protein known as NCBI RefSeq. XP_003510860.1 (SEQ ID NO:32), and further described herein. PLBD2 is also referred to in the art as putative phospholipase B-like 2 (PLBL2), 76 kDa protein, LAMA-like protein 2, PLB homolog 2, lamina ancestor homolog 2, mannose-6-phosphate protein associated protein p76, p76, phospholipase B-like 2 32 kDa form, phospholipase B-like 2 45 kDa form, or Lysosomal 66.3 kDa protein.

The term "cell" or "cell line" includes any cell that is suitable for expressing a recombinant nucleic acid sequence. Cells include those of prokaryotes and eukaryotes (single-cell or multiple-cell), bacterial cells (e.g., strains of *E*. *coli, Bacillus* spp., *Streptomyces* spp., etc.), mycobacteria cells, fungal cells, yeast cells (e.g. S. *cerevisiae, S. pombe, P. partoris, P. methanolica*, etc.), plant cells, insect cells (e.g. SF-9, SF-21, baculovirus-infected insect cells, *Trichoplusia ni*, etc.), non-human animal cells, mammalian cells, human cells, or cell fusions such as, for example, hybridomas or quadromas. In certain embodiments, the cell is a human, monkey, ape, hamster, rat or mouse cell. In certain embodiments, the cell is eukaryotic and is selected from the following cells: CHO (e.g. CHO K1, DXB-11 CHO, Veggie-CHO), COS (e.g. COS-7), retinal cells, Vero, CV1, kidney (e.g. HEK293, 293 EBNA, MSR 293, MDCK, HaK, BHK21), HeLa, HepG2, WI38, MRC 5, Colo25, HB 8065, HL-60, Jurkat, Daudi, A431 (epidermal), CV-1, U937, 3T3, L cell, C127 cell, SP2/0, NS-0, MMT cell, tumor cell, and a cell line derived from an aforementioned cell. In some embodiments, the cell comprises one or more viral genes, *e.g*. a retinal cell that expresses a viral gene (e.g. a PER.C6® cell).

### General Description

The invention is based at least in part on a recombinant host cell and cell expression system thereof that decreases expression of an endogenous host cell phospholipase protein, decreases the enzymatic function or binding ability of an endogenous host cell phospholipase protein, or has no detectable endogenous host cell phospholipase protein. The inventors discovered that disruption of PLBD2 protein expression allows for optimized and efficient purification of biopharmaceutical products in such expression systems. The invention may be employed in several ways, such as 1) utilizing gene editing tools to totally knockout phospholipase expression, whereas no measurable full-length phospholipase is expressed in the cell due to disruption of the phospholipase gene; 2) utilizing gene editing tools to eliminate or reduce enzymatic activity, whereas the phospholipase protein is expressed but rendered nonfunctional due to disruptions in the gene; and 3) utilizing gene editing tools to eliminate or reduce the ability of an endogenous host cell phospholipase protein to bind exogenous recombinant protein produced by the cell. Esterase activity was determined in protein fractions of certain antibody-producing cells. One particular esterase, phospholipase B-like (PLBD2) was determined as a contaminant in these protein fractions. Gene editing target sites were identified in a hamster PLBD2 gene that enable targeted disruption of the gene in a hamster cell (*i.e*. CHO) genome.

An optimized host cell comprising a modified PLBD2 gene is useful for the bioprocessing of high-quality proteins and is envisioned to reduce the burden of certain purification steps, thereby reducing time and cost, while increasing production yield.

The invention is also based on the specific targeting of an exogenous gene to the integration site. The methods of the invention allow efficient modification of the cell genome, thus producing a modified or recombinant host cell useful as a cell expression system for the bioprocessing of therapeutic or other commercial protein products. To this end, the methods of the invention employ cellular genome gene editing strategies for the alteration of particular genes of interest that otherwise may diminish or contaminate the quality of recombinant protein formulations, or require multiple purification steps.

The compositions of the invention, e.g. gene editing tools, can also be included in expression constructs for example, in expression vectors for cloning and engineering new cell lines. These cell lines comprise the modifications described herein, and further modifications for optimal incorporation of expression constructs for the purpose of protein expression are envisioned. Expression vectors comprising polynucleotides can be used to express proteins of interest transiently, or can be integrated into the cellular genome by random or targeted recombination such as, for example, homologous recombination or recombination mediated by recombinases that recognize specific recombination sites (*e.g*., Cre-Iox-mediated recombination).

Target sites for disruption or insertion of DNA are typically identified with the maximum effect of the gene disruption or insertion in mind. For example, target sequences may be chosen near the N-terminus of the coding region of the gene of interest whereas a DNA break is introduced within the first or second exon of the gene. Introns (non-coding regions) are not typically targeted for disruption as repair of the DNA break in that region may not disrupt the target gene. The changes introduced by these modifications are permanent to the genomic DNA of the organism.

Essentially, following identification of a target site of SEQ ID NO:33, gene editing protocols were employed to render a nonfunctional gene. Once the contaminant host cell protein is eliminated, protocols known in the art for introducing an expressible gene of interest (GOI), such as a multi-subunit antibody, along with any other desirable elements such as, *e.g*., promoters, enhancers, markers, operators, ribosome binding sites (*e.g*. internal ribosome entry sites), *etc.* are also employed.

The resulting recombinant cell line conveniently provides more efficient downstream bioprocess methods with respect to an expressible exogenous genes of interest (GOIs), since purification steps for exogenous proteins of interest are eliminated due to the absence of the contaminant host cell protein. Eliminating or refining purification procedures also results in higher amounts (titer) of the recovered protein of interest.

### Physical and Functional Characterization of Modified CHO Cells

Applicants have discovered an enzymatic activity associated with the destabilization of polysorbates (including polysorbate 20 and polysorbate 80). That activity was found to be associated with an esterase, such as a polypeptide comprising an amino acid sequence selected from the sequences listed in Table 1. A BLAST search of those peptide sequences revealed identity with a putative phospholipase B-like 2 (PLBD2, also referred to as PLBL2). PLBD2 is highly conserved in hamster (SEQ ID NO:32), mice (SEQ ID NO:34), rat (SEQ ID NO:35), human (SEQ ID NO:36), and bovine (SEQ ID NO:37). The applicants discovered that PLBD2, which copurifies under certain processes with some classes of proteins-of-interest manufactured in a mammalian cell line, has esterase activity responsible for the hydrolysis of polysorbate 20 and 80. Applicants envision that other esterase species, of which PLBD2 is an example, may contribute to polysorbate instability, depending upon the particular protein-of-interest and/or genetic/epigenetic background of the host cell. Fragments of mammalian esterase, particularly PLBD2, having identity among multiple species are exemplified in Table 1.

**TABLE 1**

| Sequence Identifier | Amino acid Sequence | Sequence Identifier | Amino acid Sequence |
|---|---|---|---|
| SEQ ID NO:1 | DLLVAHNTWNSYQNMLR | SEQ ID NO:16 | LTLLQLKGLEDSYEGR |
| SEQ ID NO:2 | LIRYNNFLHDPLSLCEACIPKP | SEQ ID NO:17 | MSMLAASGPTWDQLPPFQ |
| SEQ ID NO:3 | SVLLDAASGQLR | SEQ ID NO:18 | VTSFSLAKR |
| SEQ ID NO:4 | DQSLVEDMNSMVR | SEQ ID NO:19 | QNLDPPVSR |
| SEQ ID NO:5 | QFNSGTYNNQWMIVDYK | SEQ ID NO:20 | IIKKYQLQFR |
| SEQ ID NO:6 | QGPQEAYPLIAGNNLVFSSY | SEQ ID NO:21 | AQIFQRDQSLVEDMNSMVR |
| SEQ ID NO:7 | SMLHMGQPDLWTFSPISVP | SEQ ID NO:22 | LIRYNNFLHDPLSLCEACIPKP |
| SEQ ID NO:8 | YNNFLHDPLSLCEACIPKPNA | SEQ ID NO:23 | SVLLDAASGQLR |
| SEQ ID NO:9 | LALDGATWADIFK | SEQ ID NO:24 | DQSLVEDMNSMVR |
| SEQ ID NO:10 | LSLGSGSCSAIIK | SEQ ID NO:25 | DLLVAHNTWNSYQNMLR |
| SEQ ID NO:11 | YVQPQGCVLEWIR | SEQ ID NO:26 | YNNFLHDPLSLCEACIPKPNA |
| SEQ ID NO:12 | RMSMLAASGPTWDQLPPFQ | SEQ ID NO:27 | RMSMLAASGPTWDQLPPFQ |
| SEQ ID NO:13 | SFLEINLEWMQR | SEQ ID NO:28 | SMLHMGQPDLWTFSPISVP |
| SEQ ID NO:14 | VLTILEQIPGMVVVADADKTED | SEQ ID NO:29 | MSMLAASGPTWDQLPPFQ |
| SEQ ID NO:15 | VRSVLLDAASGQLR | SEQ ID NO:30 | VRSVLLDAASGQLR |
| | | SEQ ID NO:31 | QNLDPPVSR |

Ester hydrolysis of polysorbate 80 was recently reported (see Labrenz, S.R., "Ester hydrolysis of polysorbate 80 in mAb drug product: evidence in support of the hypothesized risk after observation of visible particulate in mAb formulations," J. Pharma. Sci. 103(8):2268-77 (2014)). That paper reported the formation of visible particles in a formulation containing IgG. The author postulated that the colloidal IgG particles formed due to the enzymatic hydrolysis of oleate esters of polysorbate 80. Although no esterase was directly identified, the author speculates that a lipase or tweenase copurified with the IgG, which was responsible for degrading the polysorbate 80. Interestingly, IgGs formulated with polysorbate 20 did not form particles and the putative esterase did not hydrolyze the polysorbate 20. The author reported that the putative lipase associated with the IgG did not affect saturated C12 fatty acid (*i.e*., laurate) (Id at 7.)

Phospholipases are a family of esterase enzymes that catalyze the cleavage of phospholipids. Each phospholipase subclass has different substrate specificity based on its target cleavage site. Phospholipase B (PLB) was identified as related to a group of prokaryotic and eukaryotic lipase proteins by virtue of the presence of a highly conserved amino acid sequence motif, Gly-Asp-Ser-Leu (GDSL) (Upton, C, and Buckley, JT. A new family of lipolytic enzymes? Trends Biochem Sci. 1995; 20:178-179). However, phospholipase B is also classified with known GDSL(S) hydrolases, and has little sequence homology to true lipases, differentiating itself structurally from phospholipases by having a serine-containing motif closer to the N-terminus than other lipases. Thus, phospholipase B-like proteins are also classified as N-terminal nucleophile (Ntn) hydrolases. Functionally, phospholipase B-like enzymes hydrolyze their target substrate (fatty acid esters such as diacylglycerophospholipids) to produce free fatty acids and ester-containing compounds (*e.g*. produces glycerophosphocholine), in a similar in manner as other phospholipases. It has been suggested that PLB-like proteins, such as phospholipase B-like protein 1 (PLBD1) and phospholipase B-like protein 2 (PLBD2), also have amidase activity, similar to other Ntn hydrolases (Repo, H. et al, Proteins 2014; 82:300-311).

Knockout of a host cell gene, such as an esterase, more particularly phospholipase B-like protein 2, may be accomplished in several ways. Rendering the phospholipase gene nonfunctional, or reducing the functional activity of the target phospholipase may be done by introducing point mutations in the phospholipase genomic sequence, particularly in the exons (coding regions). The nucleic acid sequence of SEQ ID NO:33 was identified and sequences upstream and downstream of the target site (i.e. homologous arms) may be utilized to integrate an expression cassette comprising a mutated gene by homologous recombination. Further gene editing tools are described herein in accordance with the invention.

Cell lines devoid of esterase activity, particularly PLBD2 activity, are useful for the production of therapeutic proteins to be purified and stored long term, and such cell lines solve problems associated with long term storage of pharmaceutical compositions in a formulation containing a fatty acid ester surfactant by maintaining protein stability and reducing subvisible particle (SVP) formation (see also PCT International Application No. PCT/US15/54600 filed October 8, 2015, which is hereby incorporated in its entirety into the specification).

Assays to detect enzymatic activity of a phospholipase include polysorbate degradation (putative esterase activity) measurements. Unpurified protein supernatants or fractions from CHO cells, and supernatant at each step or sequence of steps when subjected to sequential purification steps, is tested for stability of polysorbate, such as polysorbate 20 or 80. The measurement of percent intact polysorbate reported is inversely proportional to the amount of contaminant esterase activity. Other measurements for detection of esterase activity or presence of esterase in a protein sample are known in the art. Detection of esterase (*e.g.* lipase, phospholipase, or PLBD2) may be done by trypsin digest mass spectrometry.

It is hypothesized that the stability of the non-ionic detergent, *i.e*. surfactant such as polysorbate, in a protein (e.g., antibody) formulation is directly correlated to the formation of subvisible particles. Thus, degradation of the polysorbate incurs loss of surfactant activity, and therefore allows the protein to aggregate and form subvisible particles. Additionally or alternatively, the fatty acids released by the degrading sorbitan fatty acid esters may also contribute to subvisible particle formation as immiscible fatty acid droplets. Therefore, levels of subvisible particles ≥ 10 micrometers in diameter may be counted in the protein formulation in order to detect esterase activity.

Other assays for detecting phospholipase activity are known in the art. For example, glycerophospho[³H]choline formation from phosphatidyl[3H]choline following incubation of phosphatidyl[³H]choline and protein supernatant may be determined by thin-layer chromatography (following similar protocols according to Kanoh, H. et al. 1991 Comp Biochem Physiol 102B(2):367-369).

SEQ ID NO:32 disclosed herein was identified from proteins expressed in CHO cells. Other mammalian species (such as, for example, humans, rats, mice), were found to have high homology to the identified esterase. Homologous sequences may also be found in cell lines derived from other tissue types of *Cricetulus griseus,* or other homologous species, and can be identified and isolated by techniques that are well-known in the art. For example, one may identify other homologous sequences by cross-species hybridization or PCR-based techniques. In addition, variants of PLBD2, can then be tested for esterase activity as described herein. DNAs that are at least about 80% identical in nucleic acid identity to SEQ ID NO:32, or variants thereof, and having esterase activity are expected to exhibit their esterase activity on biopharmaceutical compositions and are candidates for targeted disruption in the engineered cell line. Accordingly, homologs of SEQ ID NO:32 or variants thereof, and the cells expressing such homologs are also encompassed by embodiments of the invention.

The mammalian PLBD2 sequences (nucleic acid and amino acid) are conserved among hamster, human, mouse and rat genomes. Table 2 identifies exemplary mammalian PLBD2 proteins and their degree of homology.

**TABLE 2: Amino acid identity of PLBD2 homologs**

| Mammal | SEQ ID NO: | %id Human | %id Mouse | %id Rat | %id Hamster |
|---|---|---|---|---|---|
| Hamster | 32 | 80 | 89.7 | 89 | - |
| Mouse | 34 | 80.8 | - | 92.6 | 89.7 |
| Rat | 35 | - | 92.6 | - | 89 |
| Human | 36 | - | 80.8 | - | 80 |

In certain embodiments, the targeted disruption of SEQ ID NO:33 is directed to the region selected from the group consisting of nucleotides spanning positions numbered 1-20, 10-30, 20-40, 30-50, 30-60, 30-70, 40-60, 40-70, 50-70, 60-80, 70-90, 80-100, 90-110, 110-140, 120-140, 130-150, 140-160, 150-170, 160-180, 160-180, 170-190, 180-200, 180-220, 190-210, 190-230, 200-220, 210-230, 220-240, 230-250, 240-260, 250-270, 33-62, 37-56, 40-69, 44-63, 110-139, 198-227, 182-211, and 242-271 of SEQ ID NO:33.

In another embodiment, the target sequence is wholly or partially within the region selected from the group consisting of nucleotides spanning positions numbered 1-20, 10-30, 20-40, 30-50, 30-60, 30-70, 40-60, 40-70, 50-70, 60-80, 70-90, 80-100, 90-110, 110-140, 120-140, 130-150, 140-160, 150-170, 160-180, 160-180, 170-190, 180-200, 180-220, 190-210, 190-230, 200-220, 210-230, 220-240, 230-250, 240-260, 250-270, 33-62, 37-56, 40-69, 44-63, 110-139, 198-227, 182-211, and 242-271 of SEQ ID NO:33.

In another embodiment, the esterase nucleic acid sequence is at least about 80% identical, at least about 81% identical, at least about 82% identical, at least about 83% identical, at least about 84% identical, at least about 85% identical, at least about 86% identical, at least about 87% identical, at least about 88% identical, or at least about 89% identical, at least about 90% identical, at least about 91% identical, at least about 92% identical, at least about 93% identical, at least about 94% identical, at least about 95% identical, at least about 96% identical, at least about 97% identical, at least about 98% identical, or at least about 99% identical to the sequence of SEQ ID NO:33 or target sequence thereof.

Cell populations expressing enhanced levels of a protein of interest can be developed using the cell lines and methods provided herein. The isolated commercial protein, protein supernatant or fraction thereof, produced by the cells of the invention have no detectable esterase or esterase activity. Cell pools further modified with exogenous sequence(s) integrated within the genome of the modified cells of the invention are expected to be stable over time, and can be treated as stable cell lines for most purposes. Recombination steps can also be delayed until later in the process of development of the cell lines of the invention.

### Genetically Modifying the Target Host Cell Protein

Methods for genetically engineering a host cell genome in a particular location (*i.e*. target host cell protein) may be achieved in several ways. Genetic editing techniques were used to modify a nucleic acid sequence in a eukaryotic cell, wherein the nucleic acid sequence is an endogenous sequence normally found in such cells and expressing a contaminant host cell protein. Clonal expansion is necessary to ensure that the cell progeny will share the identical genotypic and phenotypic characteristics of the engineered cell line. In some examples, native cells are modified by a homologous recombination technique to integrate a nonfunctional or mutated target nucleic acid sequence encoding a host cell protein, such as a variant of SEQ ID NO:33.

One such method of editing the CHO PLBD2 genomic sequence involves the use of guide RNAs and a type II Cas enzyme to specifically target a PLBD2 exon. Specific guide RNAs directed to exon 1 of CHO PLBD2 have been employed (see *e.g*. Table 4) in a site-specific nuclease editing method as described herein. Other methods of targeted genome editing, for example nucleases, recombination-based methods, or RNA interference, to modify the PLBD2 gene may be employed for the targeted disruption of the CHO genome.

In one aspect, methods and compositions for knockout or downregulation of a nucleic acid molecule encoding a host cell protein having 90% identical to SEQ ID NO:33, or antibody-binding variant thereof, are via homologous recombination. A nucleic acid molecule, .*e.g*. encoding an esterase of interest, can be targeted by homologous recombination or by using site-specific nuclease methods that specifically target sequences at the esterase-expressing site of the host cell genome. For homologous recombination, homologous polynucleotide molecules (*i.e*. homologous arms) line up and exchange a stretch of their sequences. A transgene can be introduced during this exchange if the transgene is flanked by homologous genomic sequences. In one example, a recombinase recognition site can also be introduced into the host cell genome at the integration sites.

Homologous recombination in eukaryotic cells can be facilitated by introducing a break in the chromosomal DNA at the integration site. Model systems have demonstrated that the frequency of homologous recombination during gene targeting increases if a double-strand break is introduced within the chromosomal target sequence. This may be accomplished by targeting certain nucleases to the specific site of integration. DNA-binding proteins that recognize DNA sequences at the target gene are known in the art. Gene targeting vectors are also employed to facilitate homologous recombination. In the absence of a gene targeting vector for homology directed repair, the cells frequently close the double-strand break by non-homologous end-joining (NHEJ) which may lead to deletion or insertion of multiple nucleotides at the cleavage site. Gene targeting vector construction and nuclease selection are within the skill of the artisan to whom this invention pertains.

In some examples, zinc finger nucleases (ZFNs), which have a modular structure and contain individual zinc finger domains, recognize a particular 3-nucleotide sequence in the target sequence. Some embodiments can utilize ZFNs with a combination of individual zinc finger domains targeting multiple target sequences. ZFN methods to target disruption of the PLBD2 gene (*e.g.* at exon 1 or exon 2) are also embodied by the invention.

Transcription activator-like (TAL) effector nucleases (TALENs) may also be employed for site-specific genome editing. TAL effector protein DNA-binding domain is typically utilized in combination with a non-specific cleavage domain of a restriction nuclease, such as Fokl. In some embodiments, a fusion protein comprising a TAL effector protein DNA-binding domain and a restriction nuclease cleavage domain is employed to recognize and cleave DNA at a target sequence within an exon of the gene encoding the target host cell protein, for example an esterase, such as a phospholipase B-like 2 protein, or other mammalian phospholipase. Targeted disruption or insertion of exogenous sequences into the specific exon of the CHO protein encoded by SEQ ID NO:33 may be done by employing a TALE nuclease (TALEN) targeted to locations within exon 1, exon 2, exon 3, etc. of the esterase genomic DNA (see Tables 3 and 4). The TALEN target cleavage site within SEQ ID NO:33 may be selected based on ZiFit.partners.org (ZiFit Targeter Version 4.2) and then TALENs are designed based on known methods (Boch J et al., 2009 Science 326:1509-1512; Bogdanove, A. J. & Voytas, D. F. 2011 Science 333, 1843-1846; Miller, J. C. et al., 2011 Nat Biotechnol 29, 143-148). TALEN methods to target disruption of the PLBD2 gene (*e.g*. exon 1 or exon 2) are also embodied by the invention.

RNA-guided endonucleases (RGENs) are programmable genome engineering tools that were developed from bacterial adaptive immune machinery. In this system-the clustered regularly interspaced short palindromic repeats (CRISPR)/CRISPR-associated (Cas) immune response-the protein Cas9 forms a sequence-specific endonuclease when complexed with two RNAs, one of which guides target selection. RGENs consist of components (Cas9 and tracrRNA) and a target-specific CRISPR RNA (crRNA). Both the efficiency of DNA target cleavage and the location of the cleavage sites vary based on the position of a protospacer adjacent motif (PAM), an additional requirement for target recognition (Chen, H. et al, J. Biol. Chem. published online March 14, 2014, as Manuscript M113.539726). CRISPR-Cas9 methods to target disruption of the PLBD2 gene (*e.g*. exon 1 or exon 2) are also embodied by the invention.

Still other methods of homologous recombination are available to the skilled artisan, such as BuD-derived nucleases (BuDNs) with precise DNA-binding specificities (Stella, S. et al. Acta Cryst. 2014, D70, 2042-2052). A single residue-to-nucleotide code guides the BuDN to the specific DNA target within SEQ ID NO:33.

Sequence-specific endonucleases, or any homologous recombination technique, may be directed to a target sequence at any one of the exons encoding PLBD2, for example in the CHO-K1 genome, NCBI Reference Sequence: NW_003614971.1, at: Exon 1 within nucleotides (nt) 175367 to 175644 (SEQ ID NO:47); Exon 2 within nt 168958 to 169051 (SEQ ID NO:48); Exon 3 within nt 166451 to166609 (SEQ ID NO:49); Exon 4 within nt 164966 to 165066 (SEQ ID NO:50); Exon 5 within nt 164564 to164778 (SEQ ID NO:51); Exon 6 within nt 162682 to162779 (SEQ ID NO:52); Exon 7 within nt 160036 to160196 (SEQ ID NO:53); Exon 8 within nt 159733 to 159828 (SEQ ID NO:54); Exon 9 within nt 159491 to 159562 (SEQ ID NO:55); Exon 10 within nt 158726 to 158878 (SEQ ID NO:56); Exon 11 within nt 158082 to 158244 (SEQ ID NO:57); or Exon 12 at nucleotides (nt) 157747 to 157914 (SEQ ID NO:58), wherein PLBD2 exons 1-12 are described on the minus strand gene and the complement of each sequence is also incorporated herewith.

Precise genome modification methods are chosen based on the tools available compatible with unique target sequences within SEQ ID NO:33 so that disruption of the cell phenotype is avoided.

### Proteins of Interest

Any protein of interest suitable for expression in prokaryotic or eukaryotic cells can be used in the engineered host cell systems provided. For example, the protein of interest includes, but is not limited to, an antibody or antigen-binding fragment thereof, a chimeric antibody or antigen-binding fragment thereof, an ScFv or fragment thereof, an Fc-fusion protein or fragment thereof, a growth factor or a fragment thereof, a cytokine or a fragment thereof, or an extracellular domain of a cell surface receptor or a fragment thereof. Proteins of interest may be simple polypeptides consisting of a single subunit, or complex multisubunit proteins comprising two or more subunits. The protein of interest may be a biopharmaceutical product, food additive or preservative, or any protein product subject to purification and quality standards.

### Host Cells and Transfection

The host cells used in the methods of the invention are eukaryotic host cells including, for example, Chinese hamster ovary (CHO) cells, human cells, rat cells and mouse cells. In a preferred embodiment, the invention provides a cell comprising a disrupted nucleic acid sequence fragment of SEQ ID NO:33.

The invention includes an engineered mammalian host cell further transfected with an expression vector comprising an exogenous gene of interest, such gene encoding the biopharmaceutical product. While any mammalian cell may be used, in one particular embodiment the host cell is a CHO cell.

Transfected host cells include cells that have been transfected with expression vectors that comprise a sequence encoding a protein or polypeptide. Expressed proteins will preferably be secreted into the culture medium for use in the invention, depending on the nucleic acid sequence selected, but may be retained in the cell or deposited in the cell membrane. Various mammalian cell culture systems can be employed to express recombinant proteins. Other cell lines developed for specific selection or amplification schemes will also be useful with the methods and compositions provided herein, provided that an esterase gene having at least 80% homology to SEQ ID NO:33 has been downregulated, knocked out or otherwise disrupted in accordance with the invention. An embodied cell line is the CHO cell line designated K1. To achieve high volume production of recombinant proteins, the host cell line may be pre-adapted to bioreactor medium in the appropriate case.

Several transfection protocols are known in the art, and are reviewed in Kaufman (1988) Meth. Enzymology 185:537. The transfection protocol chosen will depend on the host cell type and the nature of the GOI, and can be chosen based upon routine experimentation. The basic requirements of any such protocol are first to introduce DNA encoding the protein of interest into a suitable host cell, and then to identify and isolate host cells which have incorporated the heterologous DNA in a relatively stable, expressible manner.

One commonly used method of introducing heterologous DNA into a cell is calcium phosphate precipitation, for example, as described by Wigler et al. (Proc. Natl. Acad. Sci. USA 77:3567, 1980). DNA introduced into a host cell by this method frequently undergoes rearrangement, making this procedure useful for cotransfection of independent genes.

Polyethylene-induced fusion of bacterial protoplasts with mammalian cells (Schaffner et al., (1980) Proc. Natl. Acad. Sci. USA 77:2163) is another useful method of introducing heterologous DNA. Protoplast fusion protocols frequently yield multiple copies of the plasmid DNA integrated into the mammalian host cell genome, and this technique requires the selection and amplification marker to be on the same plasmid as the GOI.

Electroporation can also be used to introduce DNA directly into the cytoplasm of a host cell, for example, as described by Potter et al. (Proc. Natl. Acad. Sci. USA 81:7161, 1988) or Shigekawa et al. (BioTechniques 6:742, 1988). Unlike protoplast fusion, electroporation does not require the selection marker and the GOI to be on the same plasmid.

Other reagents useful for introducing heterologous DNA into a mammalian cell have been described, such as Lipofectin™ Reagent and Lipofectamine™Reagent (Gibco BRL, Gaithersburg, Md.). Both of these commercially available reagents are used to form lipid-nucleic acid complexes (or liposomes) which, when applied to cultured cells, facilitate uptake of the nucleic acid into the cells.

Methods for amplifying the GOI are also desirable for expression of the recombinant protein of interest, and typically involves the use of a selection marker (reviewed in Kaufman *supra*). Resistance to cytotoxic drugs is the characteristic most frequently used as a selection marker, and can be the result of either a dominant trait (*e.g*., can be used independent of host cell type) or a recessive trait (*e.g*., useful in particular host cell types that are deficient in whatever activity is being selected for). Several amplifiable markers are suitable for use in the cell lines of the invention and may be introduced by expression vectors and techniques well known in the art (*e.g*., as described in Sambrook, Molecular Biology: A Laboratory Manual, Cold Spring Harbor Laboratory, NY, 1989; pgs 16.9-16.14).

Useful selectable markers and other tools for gene amplification such as regulatory elements, described previously or known in the art, can also be included in the nucleic acid constructs used to transfect mammalian cells. The transfection protocol chosen and the elements selected for use therein will depend on the type of host cell used. Those of skill in the art are aware of numerous different protocols and host cells in order to adapt the invention for a particular use, and can select an appropriate system for expression of a desired protein, based on the requirements of the cell culture system.

Other features of the invention will become apparent in the course of the following descriptions of exemplary embodiments which are given for illustration of the invention and are not intended to be limiting thereof.

### EXAMPLES

The following examples are put forth so as to provide those of ordinary skill in the art how to make and use the methods and compositions described herein, and are not intended to limit the scope of the invention. Efforts have been made to ensure accuracy with respect to numbers used (*e.g.*, amount, temperature, *etc.*) but some experimental error and deviation should be accounted for. Unless indicated otherwise, parts are parts by weight, molecular weight is average molecular weight, temperature is in degrees Centigrade, and pressure is at or near atmospheric.

### Example 1. Targeted Disruption of an Esterase Gene in the Host Cell

To employ disruption of the target esterase gene, i.e. phospholipase B-like 2 gene, of a CHO cell origin, a Type II CRISPR/Cas system which requires at least 20 nucleotides (nt) of homology between a chimeric RNA (*i.e*. guide RNA) and its genomic target was used. Guide RNA sequences were designed for specific targeting of an exon within the CHO phospholipase B-like 2 (PLBD2) nucleic acid (SEQ ID NO:33) and are considered unique (to minimize off-target effects in the genome). Multiple small guide RNAs (sgRNA) were synthesized for use in the genome editing procedure targeting the following genomic segments of PLBD2 listed in Table 3.

**TABLE 3**

| SEQ ID NO: | SEQ ID NO:33 nucleotide numbers | SEQ ID NO:47 (nt numbers of Exon 1 at genomic locus) | genomic DNA sequence |
|---|---|---|---|
| 38 | 110-139 | 170-199 | 5'-CTGAGGTGTTGCTGAATTGCCCGGCGGGCG-3' |
| 39 | 227-198 | 82-111 | 5'-GACGCGGCGTCCAGCAGCACCGAGCGGACG-3' |
| 40 | 182-211 | 98-127 | 5'-ACCCGCCGGTCTCCCGCGTCCGCTCGGTGC-3' |
| 41 | 242-271 | 212-241 | 5'-TGGTGGACGGCATCCATCCCTACGCGGTGG-3' |
| 42 | 33-62 | 3-32 | 5'-GGCGGCCCCCATGGACCGGAGCCCCGGCGG-3' |
| 43 | 40-69 | 10-39 | 5'-CCCATGGACCGGAGCCCCGGCGGCCGGGCG-3' |

The sgRNA expression plasmid (System Biosciences, CAS940A-1) contains a human H1 promoter that drives expression of the small guide RNA and the tracrRNA following the sgRNA. Immortalized Chinese hamster ovary (CHO) cells were transfected with the plasmid encoding Cas9-H1 enzyme followed by one of the sgRNA sequences, for instance sgRNA1 (SEQ ID NO:45) or sgRNA2 (SEQ ID NO:46), designed to target the first exon of CHO PLBD2. sgRNA1 and sgRNA2 were predicted to generate a double strand break (DSB) at or around nucleotides 53 and 59 of SEQ ID NO:33, respectively. A DSB was therefore predicted to occur approx. 23 or 29 nucleotides downstream of the PLBD2 start codon. (Note that nucleotides 1-30 of SEQ ID NO:33 encode a signal peptide.) A negative control transfection was performed where the parental CHO line was transfected with the plasmid encoding Cas9-H1 enzyme without a proceeding sgRNA, or an sgRNA encoding a gene sequence not present in the CHO genome.

**TABLE 4**

| sgRNA designation | SEQ ID NO: | SEQ ID NO:33 nucleotide numbers | SEQ ID NO:47 (nt numbers of Exon 1 at genomic locus) | sgRNA (targeting vector nt sequence) |
|---|---|---|---|---|
| sgRNA1 | 45 | 37-56 | 7-26 | 5'- GCCCCCATGGACCGGAGCCC -3' |
| sgRNA2 | 46 | 44-63 | 14-33 | 5'- TGGACCGGAGCCCCGGCGGC -3' |

Following transfection, cells were cultured for 6 days in serum-free medium, and then were single cell cloned using flow cytometry. After 12 days in culture, stable clones with desirable growth properties were isolated, expanded in serum-free medium, cell pellets were collected for genotyping and clonal cell lines were banked.

Genomic DNA (gDNA) and messenger RNA (mRNA) were isolated from the clonal cell pellets and analyzed by quantitative PCR (qPCR). qPCR primers and probes were designed to overlap with the sgRNA sequence used for the double strand break targeting event, in order to detect disruption of the genomic DNA and its transcription. The relative abundance of PLBD2 gene or transcript in the candidate clones was determined using relative qPCR method, where the clones derived from the negative control transfection were used as a calibrator. See Figure 1. The qPCR primers and probes were designed to detect sequences either in the sgRNA1 or sgRNA2 position in PLBD2 exon 1. Both gDNA and RNA isolated from clone 1 failed to support qPCR amplification of PLBD2 exon 1 in either sgRNA1 or sgRNA2 regions, but amplification of the housekeeping gene, GAPDH, was detected. Based on this data, clone 1 was identified as a potential knock out of PLBD2 in which both genomic alleles of PLBD2 of exon1 were disrupted. It is noted that amplification of genomic DNA and mRNA was not detected in Clone 8 using primers overlapping with sgRNA2, however, sgRNA1 primers/probes detected genomic DNA above control values. Clone 8, and others were further analyzed in order to understand the performance of the site-directed nuclease method.

The size of the entire PLBD2 exon 1 in clone 1 was analyzed by PCR from either gDNA or RNA derived templates and compared to that amplified from the wild type CHO cells. The length of amplicon fragments was determined using Caliper GX instrument (Figure 2). Both gDNA and mRNA amplification from clone 1 resulted in a single PCR fragment which was shorter than the one amplified from the wild type control cells.

.The amplification products were sequenced, resulting in Clone 1 being identified as PLBD2 knock out, in which PLBD2 gene was found to have 11 bp deletion resulting in frameshift.

The inventors also unexpectedly identified Clone 8 as a PLBD2 knockout despite the fact that genomic DNA fragments were identified by qPCR primers overlapping with the sgRNA1 sequence. The identification of a clone that has no detectable phospholipase activity or no detectable phospholipase protein was technically challenging and time-consuming. Site-directed nuclease techniques may provide an ease-of-use, however, careful screening and elimination of false positives is necessary and still there may be unpredictable outcomes with regard to the identity of a single clone having two disrupted alleles. Surprisingly, only 1% of the clones screened using the techniques described above were identified as viable PLBD2 knockout clones. See Table 5.

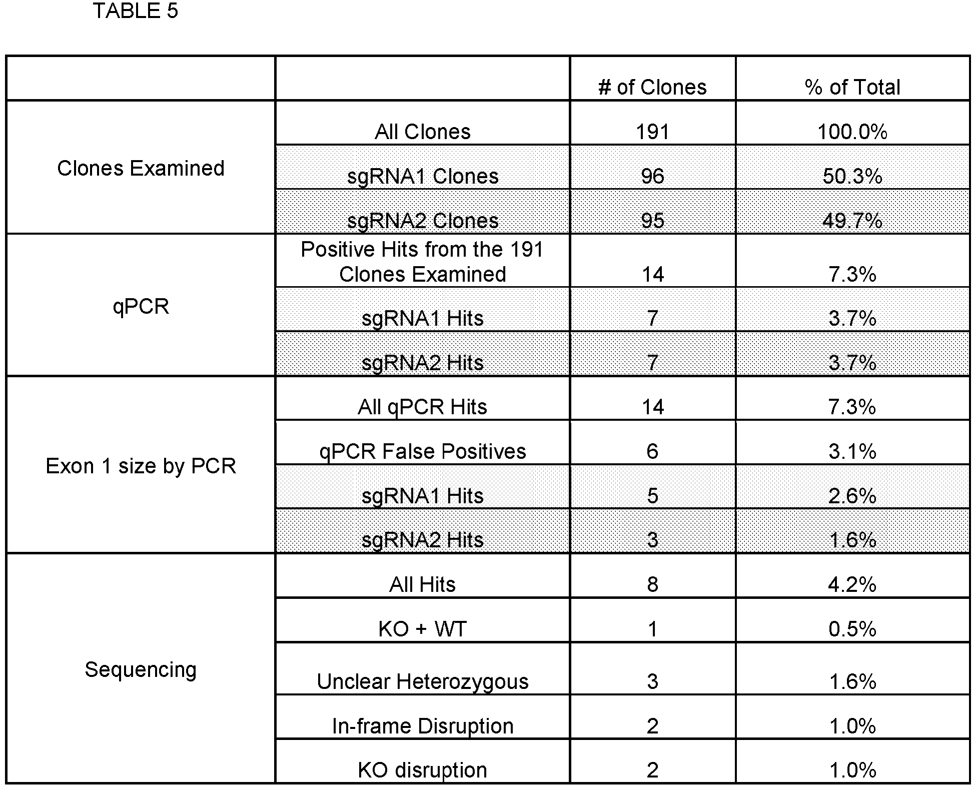

### Example 2. Introduction and Expression of a monoclonal antibody (mAb1) in the candidate clonal cell lines

Clone 1 and the wild type control host cell line were transfected with plasmids encoding the light and heavy chains of mAb1, a fully human IgG, in the presence of Cre recombinase to facilitate recombination mediated cassette exchange (RMCE) into EESYR locus (US Patent No. 7771997B2, issued August 10, 2010). The transfected cultures were selected for 11 days in serum-free medium containing 400 ug/mL hygromycin. Cells that underwent RMCE, were isolated by flow cytometry. PLBD2 knock out clone 1 and the wild type host cell line produced equivalent observed recombinant population (data not shown). The clone 1 derived isogenic cell line expressing mAb1 was designated RS001, and the mAb1 expressing cell line originated from the PLBD2 wild type host was designated RS0WT

Fed-batch production of mAb1 from RS001 or RS0WT was carried out in a standard 12 day process. The conditioned medium for each production culture was sampled at Day 0, 3, 5, 7, 10, and 13 and the Protein-A binding fraction was quantified (Figure 3). Protein titer of mAb1 from RS001 culture was comparable to that produced from RS0WT, and unexpectedly there were no observable differences in the behavior of the cells with respect to the two cultures. It cannot be predicted whether disruption of PLBD2 or any endogenous gene in a CHO host cell would have no observable deleterious effect on production of an exogenous recombinant protein, especially a therapeutic monoclonal antibody.

### Example 3. Esterase Activity Detection in unmodified CHO cells

Polysorbate 20 or polysorbate 80 degradation was measured to detect putative esterase activity in the supernatants of PLBD2 mutants. Unpurified protein supernatant from CHO cells, and supernatant taken at each step or sequence of steps when subjected to sequential purification steps, was tested for stability of polysorbate. The percent intact polysorbate reported was inversely proportional to the amount of contaminant esterase activity. Unpurified protein supernatant from CHO cells, and supernatant at each step or sequence of steps when subjected to sequential purification steps, was tested in assays measuring polysorbate degradation. The relative levels of intact polysorbate reported is inversely proportional to levels of contaminant esterase activity.

Degradation of polysorbate 20 was examined to determine the etiological agent responsible for polysorbate 20 degradation in a monoclonal antibody formulation. The buffered antibody (150 mg/mL) was separated into two fractions by 10 kDa filtration: a protein fraction, and a buffer fraction. These two fractions, as well as intact buffered antibody, were spiked with 0.2% (w/v) of super refined polysorbate 20 (PS20-B) and stressed at 45°C for up to 14 days. The study showed (Table 6, part A, columns 1-2) that the protein fraction, not the buffer fraction, had an effect on the degradation of sorbitan laurate (*i.e*., the major component of polysorbate 20), and that the degradation of polysorbate 20 was correlated with the concentration of the antibody (Table 6, part B, columns 3-4).

| TABLE 6, part A | | | TABLE 6, part B | |
|---|---|---|---|---|
| Fraction | % ester remaining (14 days at 45°C) | | Antibody concentration (mg/mL) | % ester remaining (12 days at 45°C) |
| Drug substance | 75% | | 150 | 82% |
| Protein Fraction | 75% | | 75 | 92% |
| Buffer Fraction | 100% | | 25 | 98% |

Monoclonal antibody was produced in an unmodified CHO cell and purified by different processes and the esterase activity measured by percent intact polysorbate 20, as in Table 7.

**TABLE 7**

| Process No. | Purification Steps | Percent Intact Polysorbate 20 |
|---|---|---|
| 1 | Protein A affinity capture (PA) | 54% |
| 2 | PA > cation exchange (CEX) | 25% |
| 3 | PA > CEX > anion exchange (AEX) | 86% |
| 4 | PA > CEX > hydrophobic interaction (HIC) | 90% |
| 5 | PA > AEX | 83% |
| 6 | PA > AEX > HIC | 92% |

Hydrophobic interaction chromatography (HIC) was most efficient at removing residual PLBD2. In some circumstances, a reduction in the number of purification steps and lower cost could be realized. Therefore, it was contemplated that a modified CHO cell having reduced levels of expression of phospholipase reduces the purification steps, and *e.g*. may eliminate the need for HIC purification.

### Example 4. Esterase protein abundance and activity detection in mAb1 purified from modified compared to unmodified CHO cells.

mAb1 was produced from RS001 and RS0WT and purified from the conditioned media using either PA alone, or PA and AEX chromatography The PA-purified mAB1 from RS001 and RS0WT were analyzed for lipase abundance using trypsin digest mass spectrometry. The trypsin digests of RS001 and RS0WT mAb1 were injected into a reverse phase liquid chromatography column coupled to a triple quadrupole mass spectrometer set to monitor a specific product ion fragmented from SEQ ID NO:32. In parallel, a series of PLBD2 standards were prepared by spiking in varying amounts of recombinant PLBD2 into mAb1 with no endogenous PLBD2. The signals of the experimental and control reactions were used to quantify the abundance of PLBD2 in mAb1 from RS001 and RS0WT (Figure 4). No detectable amounts of PLBD2 protein were observed in the purified samples of Clone 8-produced mAb1 when purified with PA alone (data not shown).

The present invention may be embodied in other specific embodiments.

## Claims

1. A recombinant host cell comprising a modification in a coding sequence of a polynucleotide encoding a phospholipase B-like 2 (PLBD2) protein, wherein the modification decreases the expression level of the PLBD2 protein relative to the expression level of a PLBD2 protein in a cell lacking the modification, wherein the cell does not express detectable PLBD2 protein and wherein the cell further comprises an exogenously added gene encoding a protein of interest.

2. The host cell of claim 1, wherein:
(a) all alleles of the coding sequence of the polynucleotide encoding the phospholipase B-like 2 (PLBD2) protein comprise the modification; and/or
(b) the modification comprises a nucleotide insertion or deletion within exon 1 of the coding sequence of the polynucleotide encoding the PLBD2 protein.

3. The host cell of claim 1 or 2, wherein the PLBD2 protein comprises an amino acid sequence at least 80% identical to SEQ ID NO: 32.

4. The host cell of any one of claims 1 to 3, wherein the PLBD2 protein comprises an amino acid sequence selected from the group consisting of SEQ ID NO: 32, SEQ ID NO: 34, SEQ ID NO: 35, and SEQ ID NO: 36.

5. The host cell of any one of claims 1 to 4, wherein the modification comprises a nucleotide insertion or deletion within SEQ ID NO: 47.

6. The host cell of any one of claims 1 to 5, wherein the exogenous protein of interest is selected from the group consisting of an antibody heavy chain, an antibody light chain, an antigen-binding fragment, and an Fc-fusion protein.

7. The host cell of any one of claims 1 to 6, wherein the cell produces a Protein A-binding fraction having no detectable esterase activity.

8. The recombinant host cell of any one of claims 1 to 7, wherein:
(a) the PLBD2 gene modification comprises a biallelic modification; and/or
(b) the PLBD2 gene modification comprises a deletion of 1 or more base pairs, 2 or more base pairs, 3 or more base pairs, 4 or more base pairs, 5 or more base pairs, 6 or more base pairs, 7 or more base pairs, 8 or more base pairs, 9 or more base pairs, 10 or more base pairs, 11 or more base pairs, 12 or more base pairs, 13 or more base pairs, 14 or more base pairs, 15 or more base pairs, 16 or more base pairs, 17 or more base pairs, 18 or more base pairs, 19 or more base pairs, or 20 or more base pairs of the PLBD2 gene: and/or
(c) the PLBD2 gene modification results in a frameshift of the translated PLBD2 protein.

9. The recombinant host cell of any one of claims 1 to 8, wherein the modification is a targeted disruption at a target site, wherein the target site is selected from the group consisting of the nucleotides spanning positions numbered 37-56, 44-56, 33-62, 40-69, 110-139, 198-227, 182-211, and 242-271 of SEQ ID NO: 33.

10. The recombinant host cell of any one of claims 1 to 9, wherein the recombinant host cell is a eukaryotic cell; optionally
wherein the eukaryotic cell is a mammalian cell; optionally
wherein the eukaryotic cell is selected from the group consisting of CHO K1 cell, a DXB-11 CHO cell, a Veggie-CHO cell, a COS-7 cell, a Vero cell, a CV1 cell, an HEK293 cell, a 293 EBNA cell, an MSR 293 cell, an MDCK cell, an HaK cell, a BHK21 cell, an HeLa cell, an HepG2 cell, a WI38 cell, an MRC 5 cell, a Colo25 cell, an HB 8065 cell, an HL-60 cell, a Jurkat cell, a Daudi cell, an A431 cell, a U937 cell, a 3T3 cell, an L cell, a C127 cell, a SP2/0 cell, an NS-0 cell, and an MMT cell; optionally
wherein the cell is a CHO cell.

11. A process for reducing esterase activity in a protein formulation comprising the steps of: (a) modifying a host cell to decrease or ablate the expression of phospholipase B-like 2 (PLBD2) protein, (b) transfecting the host cell with a polynucleotide encoding a protein of interest, (c) extracting a protein fraction comprising the protein of interest from the host cell, (d) contacting the protein fraction with a chromatography column selected from the group consisting of protein A affinity (PA), cation exchange (CEX), and anion exchange (AEX), (e) collecting the protein of interest from the media, and (f) combining the protein of interest with a fatty acid ester, and optionally a buffer and thermal stabilizer.

12. The process of claim 11, wherein the step of modifying a host cell to decrease or ablate the expression of phospholipase B-like 2 (PLBD2) protein comprises inserting or deleting at least one nucleotide within exon 1 of a polynucleotide encoding the PLBD2 protein.

13. The process of claim 12, wherein the polynucleotide encoding the PLBD2 protein comprises a nucleic acid sequence that is at least 80% identical to SEQ ID NO: 32.

## Patentansprüche

1. Rekombinante Wirtszelle, umfassend eine Modifikation in einer Codiersequenz eines Polynukleotids, das ein PLBD2(Phospholipase B-like 2)-Protein codiert, wobei die Modifikation das Expressionsniveau des PLBD2-Proteins relativ zum Expressionsniveau eines PLBD2-Proteins in einer Zelle, der die Modifikation fehlt, herabsetzt, wobei die Zelle kein nachweisbares PLBD2-Protein exprimiert und wobei die Zelle ferner ein exogen hinzugefügtes Gen, das ein interessierendes Protein codiert, umfasst.

2. Wirtszelle nach Anspruch 1, wobei:
(a) alle Allele der Codiersequenz des das PLBD2(Phospholipase B-like 2)-Protein codierenden Polynukleotids die Modifikation umfassen; und/oder
(b) die Modifikation eine Nukleotidinsertion oder -deletion in Exon 1 der Codiersequenz des das PLBD2-Protein codierenden Polynukleotids umfasst.

3. Wirtszelle nach Anspruch 1 oder 2, wobei das PLBD2-Protein eine Aminosäuresequenz mit einer Identität von wenigstens 80% mit SEQ ID NO:32 umfasst.

4. Wirtszelle nach einem der Ansprüche 1 bis 3, wobei das PLBD2-Protein eine aus der Gruppe bestehend aus SEQ ID NO: 32, SEQ ID NO: 34, SEQ ID NO: 35 und SEQ ID NO: 36 ausgewählte Aminosäuresequenz umfasst.

5. Wirtszelle nach einem der Ansprüche 1 bis 4, wobei die Modifikation eine Nukleotidinsertion oder -deletion in SEQ ID NO: 47 umfasst.

6. Wirtszelle nach einem der Ansprüche 1 bis 5, wobei das exogene interessierende Protein aus der Gruppe bestehend aus einer Antikörper-schwere-Kette, einer Antikörper-leichte-Kette, einem antigenbindenden Fragment und einem Fc-Fusionsprotein ausgewählt ist.

7. Wirtszelle nach einem der Ansprüche 1 bis 6, wobei die Zelle eine Protein A bindende Fraktion, die keine nachweisbare Esterase-Aktivität aufweist, produziert.

8. Rekombinante Wirtszelle nach einem der Ansprüche 1 bis 7, wobei:
(a) die PLBD2-Gen-Modifikation eine biallelische Modifikation umfasst; und/oder
(b) die PLBD2-Gen-Modifikation eine Deletion von 1 oder mehr Basenpaaren, 2 oder mehr Basenpaaren, 3 oder mehr Basenpaaren, 4 oder mehr Basenpaaren, 5 oder mehr Basenpaaren, 6 oder mehr Basenpaaren, 7 oder mehr Basenpaaren, 8 oder mehr Basenpaaren, 9 oder mehr Basenpaaren, 10 oder mehr Basenpaaren, 11 oder mehr Basenpaaren, 12 oder mehr Basenpaaren, 13 oder mehr Basenpaaren, 14 oder mehr Basenpaaren, 15 oder mehr Basenpaaren, 16 oder mehr Basenpaaren, 17 oder mehr Basenpaaren, 18 oder mehr Basenpaaren, 19 oder mehr Basenpaaren oder 20 oder mehr Basenpaaren des PLBD2-Gens umfasst; und/oder
(c) die PLBD2-Gen-Modifikation zu einer Rasterverschiebung des translatierten PLBD2-Proteins führt.

9. Rekombinante Wirtszelle nach einem der Ansprüche 1 bis 8, wobei es sich bei der Modifikation um eine gezielte Störung an einer Zielstelle handelt, wobei die Zielstelle aus der Gruppe bestehend aus den Nukleotiden, die sich über die nummerierten Positionen 37-56, 44-56, 33-62, 40-69, 110-139, 198-227, 182-211 und 242-271 von SEQ ID NO: 33 erstrecken, ausgewählt ist.

10. Rekombinante Wirtszelle nach einem der Ansprüche 1 bis 9, wobei es sich bei der rekombinanten Wirtszelle um eine eukaryontische Zelle handelt; gegebenenfalls
wobei es sich bei der eukaryontischen Zelle um eine Säugerzelle handelt; gegebenenfalls wobei die eukaryontische Zelle aus der Gruppe bestehend aus CHO-K1-Zelle, einer DXB-11-CHO-Zelle, einer Veggie-CHO-Zelle, einer COS-7-Zelle, einer Vero-Zelle, einer CV1-Zelle, einer HEK293-Zelle, einer 293-EBNA-Zelle, einer MSR-293-Zelle, einer MDCK-Zelle, einer HaK-Zelle, einer BHK21-Zelle, einer HeLa-Zelle, einer HepG2-Zelle, einer WI38-Zelle, einer MRC-5-Zelle, einer Colo25-Zelle, einer HB-8065-Zelle, einer HL-60-Zelle, einer Jurkat-Zelle, einer Daudi-Zelle, einer A431-Zelle, einer U937-Zelle, einer 3T3-Zelle, einer L-Zelle, einer C127-Zelle, einer SP2/0-Zelle, einer NS-0-Zelle und einer MMT-Zelle ausgewählt ist; gegebenenfalls wobei es sich bei der Zelle um eine CHO-Zelle handelt.

11. Verfahren zur Verringerung von Esterase-Aktivität in einer Proteinformulierung, umfassend die Schritte: (a) Modifizieren einer Wirtszelle, so dass die Expression von PLBD2(Phospholipase B-like 2)-Protein herabgesetzt oder abgesenkt wird, (b) Transfizieren der Wirtszelle mit einem Polynukleotid, das ein interessierendes Protein codiert, (c) Extrahieren einer Proteinfraktion, die das interessierende Protein umfasst, aus der Wirtszelle, (d) Inkontaktbringen der Proteinfraktion mit einer Chromatographiesäule, die aus der Gruppe bestehend aus Protein-A-Affinität (PA), Kationenaustausch (CEX) und Anionenaustausch (AEX) ausgewählt ist, (e) Sammeln des interessierenden Proteins aus den Medien und (f) Kombinieren des interessierenden Proteins mit einem Fettsäureester und gegebenenfalls einem Puffer und Thermostabilisator.

12. Verfahren nach Anspruch 11, wobei der Schritt Modifizieren einer Wirtszelle, so dass die Expression von PLBD2(Phospholipase B-like 2)-Protein herabgesetzt oder abgesenkt wird, Inserieren oder Deletieren wenigstens eines Nukleotids in Exon 1 eines das PLBD2-Protein codierenden Polynukleotids umfasst.

13. Verfahren nach Anspruch 12, wobei das das PLBD2-Protein codierende Polynukleotid eine Nukleinsäuresequenz umfasst, die eine Identität von wenigstens 80% mit SEQ ID NO: 32 aufweist.

## Revendications

1. Cellule hôte recombinante comprenant une modification dans une séquence codante d'un polynucléotide codant pour une protéine du type phospholipase B 2 (PLBD2), dans laquelle la modification diminue le niveau d'expression de la protéine PLBD2 par rapport au niveau d'expression d'une protéine PLBD2 dans une cellule dépourvue de la modification, la cellule n'exprimant pas une protéine PLBD2 détectable et la cellule comprenant en outre un gène ajouté de manière exogène qui code pour une protéine d'intérêt.

2. Cellule hôte de la revendication 1, dans laquelle :
(a) tous les allèles de la séquence codante du polynucléotide qui code pour la protéine du type phospholipase B 2 (PLBD2) comprennent la modification ; et/ou
(b) la modification comprend une insertion ou une délétion nucléotidique au sein de l'exon 1 de la séquence codante du polynucléotide qui code pour la protéine PLBD2.

3. Cellule hôte des revendications 1 ou 2, dans laquelle la protéine PLBD2 comprend une séquence d'acides aminés étant identique au moins à 80% à la SEQ ID n° : 32.

4. Cellule hôte de l'une quelconque des revendications 1 à 3, dans laquelle la protéine PLBD2 comprend une séquence d'acides aminés choisie dans le groupe constitué par les SEQ ID n° : 32, SEQ ID n° : 34, SEQ ID n° : 35 et SEQ ID n° : 36.

5. Cellule hôte de l'une quelconque des revendications 1 à 4, dans laquelle la modification comprend une insertion ou une délétion nucléotidique au sein de la SEQ ID n° : 47.

6. Cellule hôte de l'une quelconque des revendications 1 à 5, dans laquelle la protéine d'intérêt exogène est choisie dans le groupe constitué par une chaîne lourde d'anticorps, une chaîne légère d'anticorps, un fragment de liaison à l'antigène et une protéine de fusion de Fc.

7. Cellule hôte de l'une quelconque des revendications 1 à 6, la cellule produisant une fraction de liaison à la protéine A n'ayant aucune activité détectable d'estérase.

8. Cellule hôte de l'une quelconque des revendications 1 à 7, dans laquelle :
(a) la modification du gène de PLBD2 comprend une modification bi-allélique ; et/ou
(b) la modification du gène de PLBD2 comprend une délétion de 1 paire de bases ou plus, de 2 paires de bases ou plus, de 3 paires de bases ou plus, de 4 paires de bases ou plus, de 5 paires de bases ou plus, de 6 paires de bases ou plus, de 7 paires de bases ou plus, de 8 paires de bases ou plus, de 9 paires de bases ou plus, de 10 paires de bases ou plus, de 11 paires de bases ou plus, de 12 paires de bases ou plus, de 13 paires de bases ou plus, de 14 paires de bases ou plus, de 15 paires de bases ou plus, de 16 paires de bases ou plus, de 17 paires de bases ou plus, de 18 paires de bases ou plus, de 19 paires de bases ou plus ou bien de 20 paires de bases ou plus du gène de PLBD2 ; et/ou
(c) la modification du gène de PLBD2 conduit à un décalage du cadre de la protéine PLBD2 traduite.

9. Cellule hôte recombinante de l'une quelconque des revendications 1 à 8, dans laquelle la modification est une perturbation ciblée au niveau d'un site cible, dans laquelle le site cible est choisi dans le groupe constitué par les positions couvrant les nucléotides numérotés 37-56, 44-56, 33-62, 40-69, 110-139, 198-227, 182-211 et 242-271 de la SEQ ID n° : 33.

10. Cellule hôte recombinante de l'une quelconque des revendications 1 à 9, la cellule hôte recombinante étant une cellule eucaryote ; en option,
la cellule eucaryote étant une cellule de mammifère ; en option,
la cellule eucaryote étant choisie dans le groupe constitué par une cellule CHO K1, une cellule CHO DXB-11, une cellule CHO-Veggie, une cellule COS-7, une cellule Vero, une cellule CV1, une cellule HEK293, une cellule 293 EBNA, une cellule MSR 293, une cellule MDCK, une cellule HaK, une cellule BHK21, une cellule HeLa, une cellule HepG2, une cellule WI38, une cellule MRC 5, une cellule Colo25, une cellule HB 8065, une cellule HL-60, une cellule Jurkat, une cellule Daudi, une cellule A431, une cellule U937, une cellule 3T3, une cellule L, une cellule C127, une cellule SP2/0, une cellule NS-0 et une cellule MMT ; en option,
la cellule étant une cellule CHO.

11. Processus destiné à réduire l'activité d'estérase dans une formulation de protéines comprenant les étapes consistant à : (a) modifier une cellule hôte pour diminuer ou éliminer l'expression d'une protéine du type phospholipase B 2 (PLBD2), (b) transfecter la cellule hôte avec un polynucléotide codant pour une protéine d'intérêt, (c) extraire une fraction protéique, comprenant la protéine d'intérêt, à partir de la cellule hôte, (d) mettre en contact la fraction protéique avec une colonne de chromatographie, choisie dans le groupe constitué par une d'affinité pour la protéine A (PA), une échangeuse de cations (CEX) et une échangeuse d'anions (AEX), (e) recueillir la protéine d'intérêt à partir des milieux et (f) combiner la protéine d'intérêt avec un ester d'acide gras et, en option, un tampon ainsi qu'un stabilisateur thermique.

12. Processus de la revendication 11, dans lequel l'étape de modification d'une cellule hôte afin de diminuer ou d'éliminer l'expression d'une protéine du type phospholipase B 2 (PLBD2) comprend une insertion ou une délétion d'au moins un nucléotide au sein de l'exon 1 d'un polynucléotide codant pour la protéine PLBD2.

13. Processus de la revendication 12, dans lequel le polynucléotide codant pour la protéine PLBD2 comprend une séquence d'acides nucléiques qui est identique au moins à 80% à la SEQ ID n° : 32.
